(19) Europäisches Patentamt
European Patent Office
Office européen des brevets

(11) EP 1 688 146 B1

(12) **EUROPEAN PATENT SPECIFICATION**

(45) Date of publication and mention of the grant of the patent:
**18.07.2007 Bulletin 2007/29**

(51) Int Cl.:
***A61K 38/20*** *(2006.01)* ***A61K 47/12*** *(2006.01)*
***A61P 35/00*** *(2006.01)*

(21) Application number: **05250680.5**

(22) Date of filing: **07.02.2005**

(54) **Preparing aldesleukin for pharmaceutical use**

Aufbereitung von Aldesleukin zur pharmazeutischen Verwendung

Préparation d' Aldesleukin pour l 'ulilisation pharmaceutique

(84) Designated Contracting States:
**AT BE BG CH CY CZ DE DK EE ES FI FR GR HU IE IS IT LI LT LU MC NL PL PT RO SE SI SK TR**
Designated Extension States:
**AL HR LV MK**

(43) Date of publication of application:
**09.08.2006 Bulletin 2006/32**

(73) Proprietor: **Novartis Vaccines and Diagnostics, Inc.**
**Emeryville, CA 94608 (US)**

(72) Inventor: **Hora Maninder**
**c/o Chiron Corporation**
**Emeryville, CA 94608 (US)**

(74) Representative: **Marshall, Cameron John et al**
**Carpmaels & Ransford**
**43-45 Bloomsbury Square**
**London WC1A 2RA (GB)**

(56) References cited:
**US-A- 4 604 377**

- **ANONYMOUS: "PROLEUKIN IL-2 (aldesleukin) For Injection Rx only" INTERNET ARTICLE - U.S PRESCRIBING INFORMATION, [Online] XP002343428 Retrieved from the Internet: URL: http://www.proleukin.com/prescribing_i nfo/ index.aspx> [retrieved on 2005-09-01]**
- **Y.JOHN WANG AND RODNEY PEARLMAN: "Stability and Characterization of Protein and Petide Drugs: Case Histories (Chapter 8)" 1993, PLENUM PRESS , NEW YORK , XP009053205 * page 251 - page 253 ***
- **ARAKAWA TSUTOMU ET AL: "Structure and solubility of interleukin-2 in sodium dodecyl sulfate" INTERNATIONAL JOURNAL OF PEPTIDE AND PROTEIN RESEARCH, vol. 43, no. 6, 1994, pages 583-587, XP009053274 ISSN: 0367-8377**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 688 146 B1

## Description

### TECHNICAL FIELD

This invention is in the field of preparing and formulating interleukin-2 for pharmaceutical use.

### BACKGROUND ART

Interleukin-2 (IL-2) is a cytokine that promotes the growth and differentiation of T cells *in vivo* and also enhances the cytotoxicity of CD8+ T cells and natural killer cells. Administration to patients produces multiple immunological effects in a dose-dependent manner, including activation of cellular immunity with profound lymphocytosis, eosinophilia, thrombocytopenia, and the production of cytokines including TNF, IL-1 and interferon-γ. Further details of IL-2 can be found in its 'GeneCard' under accession number GC04M123831.
IL-2 has been approved for human pharmaceutical use in several countries. The recommended international non-proprietary names is ALDESLEUKIN, and a formulation is marketed by Chiron Corporation under the trade name PROLEUKIN™. The United States FDA has approved PROLEUKIN™ IL-2 for use in the treatment of adults with metastatic renal cell carcinoma (metastatic RCC) or metastatic melanoma.
As stated in its US prescribing information, PROLEUKIN™ exists as biologically active, non-covalently bound microaggregates with an average size of 27 recombinant IL-2 molecules. It is supplied as a sterile, white-to-off-white lyophilized cake in single-use vials intended for intravenous (IV) administration. When reconstituted with 1.2 ml sterile water for injection, each ml contains 18 million IU (1.1 mg) PROLELTKIN™ IL-2, 50 mg mannitol, and 0.18 mg sodium dodecyl sulfate (SDS), buffered with approximately 0.17 mg monobasic and 0.89 mg dibasic sodium phosphate to pH 7.5. Simple mixing of these ingredients does not, however, give the same microaggregated form of IL-2 as found in the PROLEUKIN™ product.
The microaggregation of IL-2 in the PROLEUKIN™ product is important for *in vivo* efficacy. The pharmacokinetic and pharmacodynamic properties of non-aggregated (free monomeric) IL-2 are very different from those of microaggregated IL-2, and the two forms have different therapeutic indexes: aggregates that are too small are cleared by the body too quickly; aggregates that are too large can be insoluble and less active. Microaggregation is thus a key feature of the PROLEUKIN™ product.
The process by which the biologically-active PROLEUKIN™ microaggregates are formed has not previously been made available to the public, even though a number of processes for preparing pharmaceutical IL-2 formulations have been described in the prior art [*e.g.* see references 1 to 30]. For instance, reference 4 discloses a process in which IL-2 is admixed with a water-soluble carrier, and a sufficient amount of SDS to ensure solubility of the IL-2 in water. The composition is lyophilised. Reference 9 in particular purports to disclose the manufacturing process for PROLEUKIN™, including details of expression, recovery, purification, formulation and finishing, but this disclosure is incomplete in several respects.
Knowing how to achieve the same active microaggregated form of IL-2 as found in the PROLEUKIN™ product would be useful for anyone wanting to make an IL-2 product similar to PROLEUKIN™. It is therefore an object of the invention to provide a process for preparing IL-2 in microaggregated form as found in the PROLEUKIN™ product, and also to provide such microaggregated IL-2. It is a further object of the invention to provide a process for preparing IL-2 to supplement the disclosure of reference 9.

### DISCLOSURE OF THE INVENTION

The process by which the PROLEUKIN™ product is prepared includes the following key steps: (i) purification of expressed IL-2 from cell culture; (ii) solubilisation of IL-2 using SDS detergent; (iii) reduction of IL-2 to monomeric form; (iv) purification, oxidation and precipitation of IL-2; (v) re-solubilisation of IL-2 using a high level of SDS detergent; (vi) purification to remove oligomeric IL-2; (vii) diafiltration to reduce levels of SDS detergent, without complete removal, to an optimal range for obtaining an appropriately microaggregated IL-2 preparation; (viii) formulation with mannitol and buffer; and (ix) lyophilisation and packaging.
Steps (i), (iv), (vi), (viii) and (ix) of this procedure are disclosed in reference 9, but steps (ii), (iii), (v) and (vii) are not, although the use of an unspecified detergent to resolubilise IL-2 prior to step (vi) is disclosed. Moreover, reference 9 suggests that SDS is added to IL-2 after purification in step (vi), when it is in fact removed.
Of the nine steps, the most critical ones by which the microaggregates are formed are (v) and (vii). Neither of downstream steps (viii) or (ix) has any significant effect on microaggregation, and the purification procedures in steps (i), (ii), (iii), (iv) and (vi) also have little effect. Once SDS is added in step (v), however, IL-2 is solubilised, and it is the subsequent reduction of SDS levels in step (vii) that causes the formation of the microaggregates present in the final PROLEUKIN™ product. Enough SDS has to be added to re-solubilise precipitated IL-2 into a substantially monomeric soluble form, and then it must be removed (but not completely) to give the desired microaggregates.

The invention therefore provides a process for preparing interleukin-2 for pharmaceutical use, comprising the steps of: (a) adding sodium dodecyl sulfate (SDS) to a composition comprising interleukin-2, to give a final SDS concentration of at least 500μg SDS per mg of IL-2; (b) removing some but not all SDS from the composition, to give a composition containing between 95-250μg SDS per mg of interleukin-2. This procedure of SDS addition and then partial removal yields optimal conditions for close interaction of IL-2 and SDS molecules to form microaggregates of IL-2 and SDS as found in the PROLEUKIN™ product and, for a given IL-2/SDS ratio, it produces a different product from that obtained by simple mixing of SDS and IL-2.

The aqueous composition arising from the process can be lyophilised for distribution, and the lyophilised material can eventually be reconstituted with an aqueous carrier for administration to patients. After reconstitution, the composition preferably has a specific turbidity (τ) less than 1.1 $cm^2/g$. and/or contains SDS/IL-2 aggregates with a mean hydrodynamic diameter of between 8 nm and 20 nm. Thus the process may further comprise the steps of lyophilising the composition and then, optionally, reconstituting the composition with an aqueous medium. The composition may also have these τ and/or hydrodynamic characteristics before the lyophilisation step.

The invention provides a composition comprising interleukin-2 and sodium dodecyl sulfate, wherein interleukin-2 and sodium dodecyl sulfate are present in the form of aggregates, and wherein the aggregates are obtainable by the processes of the invention. These compositions are suitable for pharmaceutical use, as shown by the PROLEUKIN™ product.

The invention also provides a composition comprising interleukin-2 and sodium dodecyl sulfate, wherein interleukin-2 and sodium dodecyl sulfate are present in the form of aggregates, and wherein the composition has a specific turbidity (τ) less than 1.1 $cm^2/g$. These compositions are suitable for pharmaceutical use, as shown by the PROLEUKIN™ product.

The invention also provides a composition comprising interleukin-2 and sodium dodecyl sulfate, wherein interleukin-2 and sodium dodecyl sulfate are present in the form of aggregates, and wherein the aggregates have a mean hydrodynamic diameter of between 8 nm and 20 nm. These compositions are suitable for pharmaceutical use, as shown by the PROLEUKIN™ product.

The invention also provides a composition comprising interleukin-2 and sodium dodecyl sulfate, wherein interleukin-2 and sodium dodecyl sulfate are present in the form of aggregates, and wherein the average number of interleukin-2 molecules per aggregate is between 10 and 50. These compositions are suitable for pharmaceutical use, as shown by the PROLEUKIN™ product.

The invention provides a lyophilised composition comprising interleukin-2 and sodium dodecyl sulfate, wherein the composition can be reconstituted to give an aqueous solution in which the interleukin-2 and sodium dodecyl sulfate are present in the form of aggregates, and which has a specific turbidity (τ) is less than 1.1 $cm^2/g$. Rather than, or as well as, looking at τ, the aggregates can have a mean hydrodynamic diameter of between 8 nm and 20 nm. These compositions are suitable for pharmaceutical use, as shown by the PROLEUKIN™ product.

The invention provides a process for preparing a lyophilised composition comprising interleukin-2 and sodium dodecyl sulfate, wherein: (i) the lyophilised composition can be reconstituted to give an aqueous composition in which interleukin-2 and sodium dodecyl sulfate are present in the form of aggregates, and which has a specific turbidity (τ) of less than 1.1 $cm^2/g$; and (ii) the process comprises the steps of (a) adding sodium dodecyl sulfate to a composition comprising interleukin-2, wherein sufficient sodium dodecyl sulfate is added to give monomeric solubilised interleukin-2, (b) reducing the concentration of sodium dodecyl sulfate to a level where the interleukin-2 forms aggregates with sodium dodecyl sulfate, and (c) lyophilising the composition.

The invention provides a process for preparing a lyophilised composition comprising interleukin-2 and sodium dodecyl sulfate, wherein: (i) the lyophilised composition can be reconstituted to give an aqueous composition in which interleukin-2 and sodium dodecyl sulfate are present in the form of aggregates with a mean hydrodynamic diameter of between 8 nm and 20 nm; and (ii) the process comprises the steps of (a) adding sodium dodecyl sulfate to a composition comprising interleukin-2, wherein sufficient sodium dodecyl sulfate is added to give monomeric solubilised interleukin-2, (b) reducing the concentration of sodium dodecyl sulfate to a level where the interleukin-2 forms aggregates with sodium dodecyl sulfate, and (c) lyophilising the composition.

The invention also provides a process for preparing a lyophilised composition comprising interleukin-2 and sodium dodecyl sulfate, wherein: (i) the lyophilised composition can be reconstituted to give an aqueous composition in which interleukin-2 and sodium dodecyl sulfate are present in the form of aggregates, and which has a specific turbidity (τ) less than 1.1 $cm^2/g$; and (ii) the process comprises the steps of (a) mixing sodium dodecyl sulfate and interleukin-2 to give a mixture in which sodium dodecyl sulfate is present at a first concentration, (b) removing sodium dodecyl sulfate from the mixture to reduce its concentration to a second concentration, and (c) lyophilising the composition, thereby providing said composition. The second concentration is lower than the first concentration. The first concentration will generally be at least 500μg SDS per mg of IL-2 in the mixture, and the second concentration will generally be between 95-250μg SDS per mg of IL-2.

The invention also provides a process for preparing a lyophilised composition comprising interleukin-2 and sodium dodecyl sulfate, wherein: (i) the lyophilised composition can be reconstituted to give an aqueous composition in which interleukin-2 and sodium dodecyl sulfate are present in the form of aggregates with a mean hydrodynamic diameter of between 8 nm and 20 nm; and (ii) the process comprises the steps of (a) mixing sodium dodecyl sulfate and interleukin-2 to give a mixture in which sodium dodecyl sulfate is present at a first concentration, (b) removing sodium dodecyl sulfate from the mixture to reduce its concentration to a second concentration, and (c) lyophilising the composition, thereby providing said composition. The second concentration is lower than the first concentration. The first concentration will generally be at least 500μg SDS per mg of IL-2 in the mixture, and the second concentration will generally be between 95-250μg SDS per mg of IL-2.

The invention also provides a process for preparing a lyophilised composition comprising interleukin-2 and sodium dodecyl sulfate (SDS), wherein: (i) the lyophilised composition can be reconstituted to give an aqueous composition in which interleukin-2 and SDS are present in the form of aggregates, and which has a specific turbidity (τ) less than 1.1 $cm^2/g$, and where the concentration of SDS is '*x*' μg SDS per mg of IL-2, wherein x is between 95 and 250; and (ii) the process comprises the steps of (a) mixing SDS and interleukin-2 to give a mixture in which the concentration of SDS is at least 4 times higher than '*x*', (b) removing some but not all SDS from the mixture, to give a composition in which the SDS concentration is '*x*', and (c) lyophilising the composition. The concentration is preferably at least 5 times higher than '*x*', more preferably at least 8 times higher, 10 times higher, 15 times higher, or even 20 times higher.

The invention also provides a process for preparing a composition comprising interleukin-2 and sodium dodecyl sulfate (SDS), wherein: (i) interleukin-2 and SDS in the composition are present in the form of aggregates, where the aggregates have a mean hydrodynamic diameter of between 8 nm and 20 nm, and where the concentration of SDS is '*x*' μg SDS per mg of IL-2, wherein *x* is between 95 and 250; and (ii) the process comprises the steps of (a) mixing SDS and interleukin-2 to give a mixture in which the concentration of SDS is at least 4 times higher than '*x*', (b) removing some but not all SDS from the mixture, to give a composition in which the SDS concentration is '*x*', and (c) lyophilising the composition. The concentration is preferably at least 5 times higher than '*x*', more preferably at least 8 times higher, 10 times higher, 15 times higher, or even 20 times higher.

Where a process of the invention involves reducing the SDS concentration to within the range of 95-250μg SDS per mg of IL-2, it is possible in some embodiments to reduce the concentration to below 95μg/mg (*e.g.* to between 50 and 95 μg/mg) and then to increase the concentration again to be within the 95-250μg/mg range. This increase to within the 95-250μg/mg range can conveniently take place during reconstitution of lyophilised material *e.g.* the process may involve SDS removal to <95μg/mg, followed by lyophilisation, followed by reconstitution with an aqueous SDS solution such that the SDS concentration falls within the 95-250μg/mg range.

***The detergent***

The processes of the invention involve the use of sodium dodecyl sulfate (SDS), which is also known as sodium lauryl sulfate. The formula of this anionic detergent is $CH_3(CH_2)_{11}OSO_3^-Na^+$, and it has CAS number 151-21-3 and EC number 205-788-1.

SDS is a standard reagent used to solubilise proteins and if IL-2 is present in precipitated form before SDS is added then it is used for this purpose in step (a) of the process of the invention. Starting with a composition comprising precipitated IL-2, enough SDS will be added to ensure that substantially all of the precipitated IL-2 is made soluble (except for any covalently-linked IL-2 multimers in the precipitate, which will predominantly remain precipitated even in the presence of SDS). Microaggregation of IL-2 at a concentration of 1 mg/ml is substantially absent when SDS is present at higher than about 500μg/mg, and so it is typical to add SDS to give a final SDS concentration of at least 500μg SDS per mg of IL-2 (*e.g.* at least 550, at least 600, at least 700, at least 800, at least 900, at least 1000, at least 1200, at least 1400, at least 1600, at least 1800, or at least 2000 μg SDS per mg IL-2). Using about 910 μg SDS per mg IL-2 is preferred.

The starting concentration of SDS in a sample will generally be known, because SDS is not naturally present in cells and the quantity of SDS that was added during processing of cell-derived material will have been monitored. In case the concentration of SDS is not known, however, it can be measured using conventional assay techniques, either performed on the material as a whole or on a sample of the material. For example: reference 31 discloses a capillary electrophoresis method for separation and determination of surfactants, including SDS; reference 32 discloses a single-drop method for the determination of the dodecylsulfate ion over the range 0.2-100μM using an electrode-less piezoelectric quartz crystal; reference 33 compares three methods for the convenient determination of SDS in aqueous solutions, namely titration by cetyltrimethylammonium bromide using either an ion-selective electrode or turbidity to measure the endpoint, or measurement of turbidity in the presence of myristyltrimethylammonium bromide; reference 34 discloses a method for determination of SDS, based on its interaction with fluorescent dyes such as ethidium bromide; *etc.* The preferred assay method for measuring SDS levels is a spectrophotometric assay based on reaction with acridine orange, as described in more detail in the examples and in ref. 35.

As an alternative to measuring SDS content relative to IL-2 mass, it can be measured relative to IL-2 activity.

Based on an IL-2 activity of $18 \times 10^6$ IU in 1.1mg of IL-2 (as found in PROLEUKIN™), 100μg SDS per mg IL-2 is equivalent to 6.111 μg of SDS per MIU of IL-2.

Rather than use SDS in the processes and products of the invention, it is also possible to use sodium laureth sulfate (SLS) or ammonium lauryl sulfate (ALS). Mixtures of SLS, ALS and/or SDS can also be used. In preferred embodiments of the invention, however, SDS is used.

### *Removal of detergent*

After SDS has been added to the IL-2 composition, the process of the invention involves removal of some (but not all) of the SDS, thereby providing a microaggregated form of IL-2 and SDS.

SDS can be removed by various techniques. Ion exchange chromatography can remove negatively charged SDS, and gel filtration can also be used. Most preferably, however, SDS is removed by diafiltration. In diafiltration, solvent and/or microsolutes (*e.g.* salts) are replaced by a different solvent and/or microsolutes. In general, removal and replacement occur at the same rate and the volume of the solution can thus be kept substantially constant, and so the overall effect is the replacement of original solvents/microsolutes with new solvents/microsolutes.

Diafiltration against a SDS-free buffer is the preferred way of removing SDS *e.g.* against a 10mM sodium phosphate buffer, pH 7.5.

SDS removal continues until the composition contains between 95 μg and 250 μg of SDS for every mg of interleukin-2 *e.g.* between 118-210 μg/mg, between 135-180 μg/mg, or about 160 μg/mg. The pharmacokinetic properties of IL-2 are substantially consistent over this range, and the level of SDS is not so high as to give rise to toxicity concerns. As the SDS concentration drops below about 95 μg/mg, however, the size of microaggregates rises sharply (Figure 1), rising from an average of ~60 IL-2 molecules per aggregate at 95μg/mg to an average of ~180 at 75μg/mg.

Addition and then incomplete removal of SDS results in the biologically-active IL-2 microaggregates that are found in the PROLEUKIN™ product. Simple mixing of SDS and IL-2 at the concentrations found in the PROLEUKIN™ product does not give microaggregates. Reference 36 describes a study where SDS was added to IL-2 preparations containing, like the PROLEUKIN™ product, 1.1 mg of IL-2. SDS was found to precipitate IL-2 at detergent concentrations between 0.02%-0.05%. In contrast, IL-2 does not precipitate at these SDS concentrations during SDS removal according to the invention - even as SDS decreases from 1200μg/mg (about 0.1% SDS based on 1.1 mg IL-2) down to 70μg (<0.01%), IL-2 precipitation is not seen.

During its removal, SDS concentration can be determined as described above. Where the removal method is standardised and reproducible then monitoring of SDS levels during SDS removal may not be necessary, as the same result can generally be expected each time the procedure is performed (although final confirmatory measurements may be performed). Where a removal method is variable, however, or where regular monitoring is required for other reasons, regular or continuous measurement of SDS levels can be performed until the desired final concentration is reached.

### *Further process steps*

As mentioned above, the key process steps for formation of IL-2/SDS microaggregates are (a) the addition and then (b) subsequent removal of SDS. While these two steps give IL-2 in a form suitable for use as an active ingredient in a pharmaceutical, they alone are not suitable for preparing a final pharmaceutical composition, and other steps are required. These steps may occur before steps (a) and (b), after steps (a) and (b), and/or between steps (a) and (b).

The first step in a process for preparing IL-2 for pharmaceutical use will generally be expression of the protein. This will generally involve the use of a host cell containing a gene encoding the IL-2 of interest, such as a bacterial host cell, a yeast host cell or a mammalian host cell. As an alternative to the recombinant route, however, it is also possible to activate or up-regulate expression of an endogenous IL-2 gene in a host cell's genome (*e.g.* by promoter activation), or to purify the hormone from a natural source (*e.g.* from blood).

Expression of IL-2 in a recombinant *E.coli* is a preferred route for obtaining IL-2, with IL-2 forming inclusion bodies. Suitable *E.coli* were deposited under the provisions of the Budapest Treaty at the ATCC in 1984 with accession numbers 39452 and 39626.

Processes for growing, harvesting, disrupting, or extracting IL-2 from various cell types are known in the art *e.g.* see references 19-30 and 37-41.

After expression, or when starting with expressed material, an initial step of IL-2 purification will be used. If IL-2 is present within cells then the purification step will involve cell disruption (*e.g.* by osmotic shock, homogenisation, sonication, *etc.*) in order to release the protein; if IL-2 is already present in solution (*e.g.* it was secreted after expression) then disruption is not required. Inactivation of living cells may also be performed, as may centrifugation. At the end of the initial purification step, IL-2 will typically be in precipitated form, particularly if being purified from inclusion bodies, but will have been separated from cellular debris to give a IL-2-rich paste for subsequent treatment.

After preparation of precipitated purified IL-2, or when starting with precipitated material, IL-2 can be solubilised in order to separate it from other host proteins *etc.* Solubilisation can be achieved by adding a detergent such as SDS, preferably to give a final concentration of between 4 and 4.5%. This can conveniently be achieved by adding a 5% SDS solution.

After solubilisation, or when starting with solubilised material, IL-2 can be reduced, in order to break any disulfide bridges in the protein, and in particular to break any intermolecular disulfide bridges. Any covalent IL-2 aggregates formed by intermolecular disulfide bridging are therefore converted into monomeric form. Reducing agents commonly used to reduce disulfide bridges include β-mercaptoethanol and dithiothreitol (DTT). The presence of detergent will generally be maintained in order to ensure IL-2 solubility.

After reduction, or when starting with reduced material, IL-2 can then be re-oxidised to give monomeric IL-2 with intramolecular disulfide bridges.

After re-oxidation, or when starting with re-oxidised material, IL-2 can then be further purified in order to remove reagents such as the oxidising agent, to remove endotoxin, to remove host cell DNA and/or proteins, to remove IL-2 isoforms or conformers (*e.g.* oxidized forms of IL-2), *etc.* This purification can conveniently be achieved using reverse phase HPLC.

After RP-HPLC purification, or when starting with soluble RP-HPLC-purified material, IL-2 can be precipitated *e.g.* by adding 0.8N sodium hydroxide. As well as precipitating the IL-2, this allows the protein to be separated from any organic solvents used during the RP-HPLC. Precipitated material can conveniently be collected by centrifugation.

Material obtained after expression, purification, solubilisation, reduction, oxidation, RP-HPLC and precipitation (as described above) is ideal for resolubilisation and diafiltration according to the process of the invention, although IL-2 prepared by other purification routes can also be used. Typically, however, the IL-2 will be in a precipitated form before SDS is added in step (a) of the process of the invention.

As mentioned above, covalently-linked IL-2 multimers remain precipitated even in the presence of SDS. As these multimers are much larger than the SDS-solubilised IL-2 proteins (because they are non-dissociable) they can conveniently be removed between steps (a) and (b) (*i.e.* after SDS addition but before diafiltration) by gel filtration. The presence of SDS should be maintained through the gel filtration in order to prevent precipitation of the IL-2.

After diafiltration to remove SDS and form microaggregates, an IL-2 solution may be subjected to one or more of the following steps: dilution to give a desired final dose; sterilisation, typically by sterile filtration *e.g.* through a 0.22μm filter; pharmaceutical formulation (see below); lyophilisation; packaging; *etc.*

### *Microaggregates*

The result of SDS solubilisation and diafiltration by the process of the invention is the formation of biologically-active microaggregates of SDS and IL-2, as seen in the PROLEUKIN™ product.

The precise molecular structure of the SDS/IL-2 microaggregates has not been determined, but they are believed to be micellar or micelle-like structures. At a SDS concentration of ~160μg/mg, a typical micelle contains ~30 IL-2 molecules and ~150 SDS molecules. The SDS and IL-2 in the microaggregates interact non-covalently, and the hydrophobic tail of IL-2 (residues 121-133) may interact with the alkyl backbone of SDS to form the micelles. Some SDS may remain free in solution.

The microaggregates can be detected by light scattering, and this technique also allows their size to be measured (see below). The relationship between SDS concentration and average microaggregate size, as measured by classical light scattering, has been empirically assessed, as shown in Figure 1.

As SDS removal proceeds, a concentration is reached below which the size of the aggregates starts to increase exponentially (see Figure 1). According to the invention, SDS is removed until its concentration is between 95 μg and 250 μg per mg of IL-2. Over this range, the specific turbidity (τ) is between ~0.05 and ~1.00 $cm^2/g$, which implies an average of fewer than 50 IL-2 molecules per aggregate. At 160μg SDS per mg IL-2 the specific turbidity (τ) is about 0.48 (about 27 IL-2 molecules).

The skilled person will understand that a given composition of SDS/IL-2 according to the invention will rarely, if ever, contain a single species of microaggregate. Rather, light scattering measurements reveal that the microaggregates have a variety of sizes falling within a distribution, and measurements can reveal a mean size. Where a composition comprises microaggregates with a stated average size, therefore, there will generally be some smaller microaggregates and some larger microaggregates.

Preferred compositions of the invention include SDS/IL-2 microaggregates such that the composition has a specific turbidity (τ) of between 0.3 and 0.6 *e.g.* between 0.4 and 0.5, or about 0.45. These compositions apply in particular to compositions that have been reconstituted after lyophilisation, and the value of τ can be lower before lyophilisation and reconstitution (Figure 14).

Preferred compositions of the invention include SDS/IL-2 microaggregates with a hydrodynamic diameter between 11 nm and 13 nm.

Preferred microaggregates of the invention contain between 10 and 50 IL-2 molecules *e.g.* between 20 and 35, and preferably about 27.

In preferred compositions, substantially all of the IL-2 is present in the form of microaggregates. Compositions may be substantially free from monomeric IL-2.

### *Specific turbidity (τ)*

Because SDS/IL-2 microaggregates of the invention are affected by prevailing conditions, techniques such as size exclusion chromatography and analytical centrifugation cannot be used to assess their size. Instead, classical (or static) light scattering (CLS) can be used to detect and analyse microaggregates by measuring their turbidity. Dynamic light scattering (DLS) can also be used. Measurements can be performed on specific instrumentation, or can be performed on a fluorimeter with excitation and emission monochrometers set at the same wavelength (*e.g.* 467nm from a xenon lamp, or 488nm from an argon lamp, both of which are remote from wavelengths that are absorbed by the analyte) and/or using suitable filters (*e.g.* yellow filters), such that the fluorimeter acts as a 90° CLS photometer.

When measuring light scattering, a standard spectroscopic parameter is the 'Rayleigh ratio' ($R_\Theta$). When scattered light is measured at 90° relative to incident light (*i.e.* when Θ=90°), the Rayleigh ratio is known as $R_{90}$ [*e.g.* see ref. 42], and 90° scattering is used for turbidity measurements.

Turbidity measurements (scattered intensity at 90°) can be normalised relative to optical grade toluene *i.e.* the turbidity measurement for a standard toluene sample is divided by the $R_{90}$ of toluene. By normalising in this way then the absolute value of the scattered light intensity can be determined.

For IL-2 microaggregates of the invention, turbidity can be measured at 467nm or 488nm, 25°C. Prior to assessing turbidity, compositions can be centrifuged or filtered to remove large particles (*e.g.* precipitated protein) which will otherwise distort data. When centrifuged, IL-2 compositions of the invention preferably retain at least 97% (*e.g.* ≥98%, ≥99% or more) of total IL-2 in the supernatant.

Turbidity measurements for SDS/IL-2 mixtures can be converted into absolute specific turbidity (τ) values as follows:

$$\tau = \frac{T_t - 0.98T_i}{T_i} \times \frac{3.2 \times 10^{-2}}{C}$$

where:

- $T_i$ is the turbidity intensity of the IL-2 composition,
- $T_t$ is the turbidity intensity of a toluene control,
- $C$ is the concentration of IL-2 in the IL-2 composition.

When $T_i$ and $T_t$ are measured in $cm^{-1}$ and $C$ is measured in g/ml (*i.e.* $g/cm^3$) then the units of τ are $cm^2/g$. This is the preferred measure for assessing turbidity of IL-2 compositions of the invention.

Compositions of the invention may have a specific turbidity (τ) less than 1.1 $cm^2/g$, but preferably greater than 0.1 $cm^2/g$. Preferred compositions have τ less than 0.7 $cm^2/g$. The compositions of the invention preferably have τ in the range 0.3-0.6 $cm^2/g$.

### *Dynamic light scattering*

As described in reference 43, in particular chapter 10, dynamic light scattering (DLS) can be used to determine the hydrodynamic diameter (or radius) of particles within a solution. SDS/IL-2 aggregates of the invention can be detected by dynamic light scattering (DLS). Using DLS, preferred aggregates have a number mean hydrodynamic diameter of between 8 nm and 20 nm, preferably between 10 nm and 15 nm, more preferably between 11 nm and 13 nm, and most preferably about 12 nm. As shown in Figure 17, aggregates will have diameters distributed around this mean.

In practice, some larger particles may be detected by DLS (*e.g.* the 54nm peak in Figure 17), but the hydrodynamic diameter referred to above is that of the main DLS peak, which will typically represent at least 90% by number (*e.g.* at least 95%, at least 97%, at least 99%, at least 99.5% or more) in the DLS spectrum. Thus at least 90% by number of the particles detected by DLS will have the specified number mean.

Interpretation of DLS data can be complicated by the presence of slowly-varying components, such as dust particles or mixing phenomena. To avoid these complications, IL-2 compositions of the invention for DLS measurement are preferably free from particles with a diameter greater than about 1μm (*e.g.* dust-free), and this may be achieved by the use of suitable filters *e.g.* 0.22μm filters. The compositions should also be well-mixed prior to DLS measurement. If DLS measurement reveals a slowly varying component in the composition, the results should be rejected and the measurement should be repeated on a new sample.

***The interleukin-2***

IL-2 is a well-known protein, and processes and products of the invention can use any suitable form of IL-2. The IL-2 will usually be human IL-2, or will be an IL-2 derivative that retains biological activity in humans.

IL-2 is naturally translated in humans as a precursor protein (*e.g.* the 153-mer in GenBank under accession number NP_000577.2). The precursor is cleaved (*e.g.* after Ser-20 in NP_000577.2) to give a mature product with a N-terminal alanine (SEQ ID NO: 4).

When expressed in *E.coli,* it is usual to replace the natural signal peptide with a single N-terminus methionine residue (*e.g.* SEQ ID NOS: 3 and 4). When expressed, this methionine is cleaved without intervention, leaving the natural human N-terminus residue of alanine (*e.g.* SEQ ID NO: 3 is converted to SEQ ID NO: 1).

The IL-2 in PROLEUKIN™ lacks the natural Ala-1 residue. The absence of the usual N-terminus alanine means that the IL-2 in PROLEUKIN™ is formally referred to as a "des-alanyl-1" form of IL-2. Des-alanyl-1 forms of IL-2 are preferred for use with the invention.

The IL-2 in PROLEUKIN™ is also designed to have two internal cysteine residues, rather than the natural three. This reduces the number of possible pairings of cysteine residues (and thus intramolecular disulfide bridges) from three to one [46], and improves the outcome of protein re-folding and storage stability. The substitution of a serine residue for the natural Cys-125 residue [44] means that the IL-2 in PROLEUKIN™ is formally referred to as a "serine-125" form of IL-2. Serine-125 forms of IL-2 are preferred for use with the invention. The three-dimensional crystal structure of a

Ser-125 form of human IL-2 expressed in *E.coli* is available in the structure databases under accession '3INK'.

Other substitutions within an IL-2 amino acid sequence can be made, particularly those in which the substitution is conservative *i.e.* those which take place within a family of amino acids that are related in their side chains. Amino acids can be divided into four families: (1) acidic - aspartate, glutamate; (2) basic - lysine, arginine, histidine; (3) non-polar - alanine, valine, leucine, isoleucine, proline, phenylalanine, methionine, tryptophan; (4) uncharged polar - glycine, asparagine, glutamine, cysteine, serine threonine, tyrosine. Phenylalanine, tryptophan, and tyrosine are sometimes classified as the family of aromatic amino acids. For example, it is reasonably predictable that an isolated replacement of leucine with isoleucine or valine, an aspartate with a glutamate, a threonine with a serine, or a similar conservative replacement of an amino acid with a structurally related amino acid, will not have a major effect on the biological activity. The skilled person can readily determine regions of IL-2 that can tolerate change, based on natural variation, alignments of active forms of proteins, *etc.,* and substituted proteins can be tested for biological activity *e.g.* by standard 86/504.

Guidance as to regions of the IL-2 protein that can be altered either via residue substitutions, deletions, or insertions can also be found in the art (*e.g*. structure/function relationships and/or binding studies), such as references 45 to 52, *etc*. Reference 53 discloses a variety of IL-2 mutants, including: Asn-26-Gln; Trp-121-Phe; deletion of all residues following Arg-120; and the Met-1 forms thereof. Reference 44 discloses IL-2 with deletion or substitution of Cys-125, with or without N-terminal Ala. Reference 54 discloses substitution or deletion of residues including number 2, 3, 4, 5, 6, 104 and 125. Met-104 is susceptible to oxidation, so mutation of this residue (*e.g.* substitution with Glu, Val, Ala, *etc.*) can improve stability [55]. Reference 56 discloses IL-2 substitutions at Asp-20 (with His or lie), at Asn-88 (with Arg, Gly, or Ile), and/or at Gln-126 (with Leu or Glu), which reportedly have reduced toxicity and selectivity for high affinity IL-2 receptors. Reference 57 discloses substitutions Arg-38-Ala and Phe-42-Lys in order to reduce toxicity for use during immunotherapy. Reference 58 discloses mutation of a native $Xaa^1$-Asp-$Xaa^2$ tripeptide sequence, where $Xaa^1$ is Leu, Ile, Gly or Val, and $Xaa^2$ is Val, Leu or Ser, in order to reduce vascular leak. Reference 59 discloses peptides with a N-terminus methionine fused to the first 30 residues of IL-2, optionally with an Asp-20-Lys substitution, which are said to retain IL-2-like activity.

Other known substitutions include: T7A, T7D, T7R, KSL, K9A, K9D, K9R, K9S, K9V, K9W, T10K, T10N, Q11A, Q11R, Q11T, E15A, H16D, H16E, L19D, L19E, D20E, I24L, K32A, K32W, N33E, P34E, P34R, P34S, P34T, P34V, K35D, K351, K35L, K35M, K35N, K35P, K35Q, K35T, L36A, L36D, L36E, L36F, L36G, L36H, L361, L36K, L36M, L36N, L36P, L36R, L36S, L36W, L36Y, R38D, R38G, R38N, R38P, R38S, L40D, L40G, L40N, L40S, T41E, T41G, F42A, F42E, F42R, F42T, F42V, K43H, F44K, M461, E61K, E61M, E61R, E62T, E62Y, K64D, K64E, K64G, K64L, K64Q, K64R, P65D, P65E, P65F, P65G, P65H, P651, P65K, P65L, P65N, P65Q, P65R, P65S, P65T, P65V, P65W, P65Y, L66A, L66F, E67A, L72G, L72N, L72T, F78S, F78W, H79F, H79M, H79N, H79P, H79Q, H79S, H79V, L80E, L80F, L80G, L80K, L80N, L80R, L80T, L80V, L80W, L80Y, R81E, R81K, R81L, R81M, R81N, R81P, R81T, D84R, S87T, N88D, N88H, N88T, V91A, V91D, V91E, V91F, V91G, V91N, V91Q, V91W, L94A, L94I, L94T, L94V, L94Y, E95D, E95G, E95M, T102S, T102V, M104G, E106K, Y107H, Y107K, Y107L, Y107Q, Y107R, Y107T, E116G, N119Q, T123S, T123C, Q126I, and Q126V.

Biologically active variants of IL-2 will generally have at least about 70%, (*e.g.* ≥80%, ≥90%, ≥95%, ≥98%, ≥99%) amino acid sequence identity to the amino acid sequence of the reference IL-2 polypeptide molecule, such as native human IL-2 discussed above. A variant may, for example, differ by as few as 1 to 15 amino acid residues, as few as 1 to 10 residues, such as 6-10, as few as 5, as few as 4, 3, 2, or even just 1 amino acid residue.

C-terminal extension of IL-2 has been reported [60]. IL-2 used with the invention preferably does not have any such C-terminal extension.

Other forms of IL-2 that can be used include non-human sequences. For example, the IL-2 may be from: rhesus monkey (*Mucucu mulatto;* GenBank Accession P51498); olive baboon (*Papio unubis;* GenBank Q865Y1); sooty mangabey (*Cercocebus torquatus atys;* P46649); crab-eating macaque (*Macaca fascicularis;* Q29615); common gibbon (*Hylobates lar;* ICGI2); common squirrel monkey (*Saimiri sciureus;* Q8MKH2); cow (*Bos taurus;* P05016 or NP-851340; mature sequence represented by residues 24-158 of GenBank Accession No. NP-851340); water buffalo (*Bubalus bubalis;* Q95KP3); horse (*Equus caballus;* P37997); goat (*Capra hircus;* P36835); sheep (Ovis *aries;* P19114); pig (*Sus scrofu;* P26891); red deer (*Cervus elaphus;* P51747); dog (*Canis familiaris;* Q29416); cat (*Felis catus;* 407885); rabbit (*Oryctolagus cuniculus;* 077620); killer whale (*Orcinus orca*; 097513); northern elephant seal (*Miroungu angustirostris;* 062641); domestic mouse (*Mus musculus;* NP-032392); western wild mouse (*Mus spretus;* 408867); Norway rat (*Rattus norvegicus;* P17108); Mongolian gerbil (*Meriones unguiculatus*; Q08081). Any of the variant forms disclosed in these GenBank entries may also be used. These GenBank entries generally disclose the precursor sequences which, as described above for the human sequence, are processed to give a final biologically-active form *e.g.* by cleavage of the first 20 amino acids.

Des-alanyl-1, serine-125 forms of human IL-2 are particularly preferred for use with the invention. The most preferred form of IL-2 has the 132-mer amino acid sequence shown in Figure 2 (SEQ ID NO: 1), as found in PROLEUKIN™. Compositions are preferably free from IL-2 that does not have N-terminal Ala. The Figure 2 protein can be expressed using the nucleic acid sequence shown in Figure 4 (SEQ ID NO: 2), after cleavage of the translated N-terminal methionine.

The IL-2 in the PROLEUKIN™ product is non-glycosylated, as a consequence of the *E.coli* expression host. Non-glycosylated IL-2 is preferred for use with the invention.

Methods for detecting the presence of IL-2 in a composition and for quantifying it are known in the art. Immunological detection using anti-IL-2 antibodies (*e.g.* ELISA) is the most common detection method, and these methods can also be used quantitatively. Other quantitative methods include amino acid analysis, Lowry protein assay with a regulatorily-approved protein standard, HPLC assays, and intrinsic ultraviolet absorbance measurements.

The standard quantitative measure for IL-2 is the International Unit (IU) which is based not on mass of protein but on activity in a biological assay. According to standard 86/504, 1 IU is the amount of IL-2 that produces 50% of maximal proliferation of an established IL-2-dependent cell line [61] CTLL-2. Quantification of IL-2 activity in other preparations is achieved by comparing dose response curves of the preparation with available standards (*e.g.* the WHO IL-2 standard ampoule, which contains 100 IU/ml after reconstitution) using a parallel line analysis, or by computerized software such as the ALLFIT program [62,63]. In practice, when manufacture is standardised then a conversion between drug weight and IUs is possible. For the PROLEUKIN™ product, for instance, the conversion is 1.1 mg IL-2 equals $18x10^6$ IU.

Activity can be converted to specific activity (*i.e.* activity per unit mass of IL-2 *e.g.* IU/mg) by dividing it by the mass of IL-2 present. A preferred composition of the invention has a specific activity of between 16 and 17 MIU/mg.

### *Pharmaceutical compositions*

The processes of the invention give IL-2/SDS microaggregates that are suitable for pharmaceutical use in humans, and so the invention provides a pharmaceutical composition comprising IL-2/SDS microaggregates of the invention. Rather than simply using microaggregates straight from diafiltration, however, they will typically be formulated before administration to patients.

For example, the microaggregates may be diluted in order to give a standard concentration, while maintaining the IL-2/SDS ratio. Dilution to give an IL-2 concentration of about 1.1 mg/ml is typical.

The microaggregates (*e.g.* after dilution) may be mixed with stabilisers, particularly if they are to be lyophilised. Addition of sugars (*e.g.* sucrose, trehalose) is typical to give stability during lyophilisation, and a preferred stabiliser is mannitol. This sugar alcohol can be added to give a final concentration of between 40 and 50 (*e.g.* about 45.5) mg mannitol per mg of IL-2 (50mg for 1.1 mg of IL-2). Human serum albumin (preferably recombinant) can also be added as a stabiliser. Mixtures of sugars can also be used *e.g.* sucrose & mannitol, trehalose & mannitol, *etc.*

'Buffer may be added to the microaggregate composition *e.g.* a Tris buffer, a histidine buffer, a glycine buffer or, preferably, a phosphate buffer (*e.g.* containing sodium dihydrogen phosphate and disodium hydrogen phosphate). Addition of buffer to give a pH between 7.2 and 7.8 is preferred, and in particular a pH of about 7.5.

Compositions comprising the microaggregates can be sterilised, typically by filter sterilisation *e.g.* through a 0.22μm filter. This can be preceded by filtration through a filter with larger pores.

Aqueous compositions comprising the microaggregates can be packaged directly into syringes, ready for injection. They can also be packaged into nebulisers, ready for inhalation.

Aqueous compositions comprising the microaggregates can be lyophilised prior to distribution. Aqueous or lyophilised material can be packaged into containers (*e.g.* vials), which can then be hermetically sealed. Lyophilisation will typically take place within vials, with sealing after lyophilisation. Lyophilised material equivalent to $22x10^6$ IU of IL-2 may be packaged in a single vial as a unit dose, for reconstitution in a final aqueous volume of about 1.1ml, to give about $18x10^6$ IU/ml. Fractional amounts (e.g. ½, ⅓, ¼ $^1/_5$, ⅛) thereof may also be packaged as unit doses. In particular, unit doses of about $9x10^6$ IU, about $4.5x10^6$ IU and about $14x10^6$ IU can be used.

For reconstitution after lyophilisation, sterile water for injection may be used. It is also possible to reconstitute a lyophilised cake with an aqueous composition comprising human serum albumin (preferably recombinant). Reconstitution into an aqueous volume of between 1.0 ml and 1.2 ml is preferred (*i.e.* IL-2 can be diluted to give the same concentration as before lyophilisation). This reconstituted material can then be diluted aseptically into a larger volume of liquid for intravenous infusion *e.g.* into about 50ml of a D5W solution (5% Dextrose Injection). Specific turbidity remains substantially constant during D5W dilution, until the IL-2 concentration drops below about 0.1mg/ml. Dilution can take place in various containers *e.g.* glass bottles, plastic (*e.g.* polyvinyl chloride) bags, *etc.* Dilution to give a final IL-2 concentration outside the range 30-70 μg/ml should be avoided. Conditions that cause changes in microaggregation should be avoided *e.g.* reconstitution with Bacteriostatic Water for Injection or with 0.9% Sodium Chloride Injection.

A first preferred pharmaceutical composition is thus an aqueous composition with a pH of about 7.5, comprising 1.1 mg/ml of IL-2, 0.18mg SDS, 50mg/ml mannitol and phosphate buffer, where the IL-2 and SDS form microaggregates in which the average number of IL-2 molecules per aggregate is between 10 and 50. These compositions can be reconstituted from lyophilised material.

A second preferred pharmaceutical composition is the product of diluting the first preferred pharmaceutical composition into a D5W solution.

Preferred compositions are free from preservative, free from antibiotics, and free from proteins other than IL-2 (including free from HSA) *i.e.* these materials are undetectable.

***Pharmaceutical methods and uses***

The invention provides a method for treating a patient, comprising administering a pharmaceutical composition of the invention to the patient. The patient is preferably a human, and may be a child (*e.g.* a toddler or infant), a teenager or an adult, but will generally be an adult.

The invention also provides SDS/IL-2 microaggregates of the invention for use as a medicament.

The invention also provides the use of microaggregates of the invention in the manufacture of a medicament for treating a patient.

These uses, methods and medicaments are preferably for the treatment of a cancer, particularly those with solid tumours, such as renal cell carcinoma or melanoma, which cancer may be metastatic. The uses, methods and medicaments can also be used for the treatment of patients who have received a tissue or organ transplant, or who are ready to receive one. The uses, methods and medicaments can also be used for the treatment of patients who are infected with HIV, including those with and without AIDS.

Patients for treatment with the invention, therefore, will have been diagnosed with a cancer. Patients who should not be treated according to the invention, however, may include: (1) patients with hypersensitivity to IL-2 or any other component of the pharmaceutical composition; (2) patients with a performance status of ECOG (Eastern Cooperative Oncology Group) ≥2; (3) patients with a simultaneous presence of a performance status of ECOG >1 and more than one organ with metastatic disease sites and a period of < 24 months between initial diagnosis of primary tumour and the date the patient is evaluated for IL-2 treatment; (4) patients with a significant history or current evidence of severe cardiac disease; (5) patients with evidence of active infection requiring antibiotic therapy; (6) patients with a $PaO_2 < 60$ mm Hg during rest; (7) patients with pre-existing severe major organ dysfunction; and (8) patients with central nervous system metastases or seizure disorders, with the exception of patients with successfully treated brain metastases.

Methods for checking the efficacy of therapeutic IL-2 treatment are known in the art.

The serum half-life curves of IL-2 in humans following intravenous bolus administration or infusion can be described as bi-exponential. For a bolus, the $T_{½}\alpha$ is 8 minutes and the $T_{½}\beta$ is 88 minutes; for an infusion, the $T_{½}\alpha$ is 15 minutes and the $T_{½}\beta$ is 96 minutes. Observed serum levels are proportional to the IL-2 dose.

Characteristics of suitable medicaments are given above in the section entitled 'Pharmaceutical compositions'. These compositions will generally be administered directly to a patient. Direct delivery may be accomplished by parenteral injection (*e.g.* intravenously, subcutaneously, intraperitoneally, intramuscularly, or to the interstitial space of a tissue), or by rectal, oral, vaginal, topical, transdermal, intranasal, ocular, aural, pulmonary or other mucosal administration. In general, however, IL-2 is administered by intravenous injection (*e.g.* continuous infusion or bolus) or by subcutaneous injection (*e.g.* continuous infusion or bolus).

IL-2 dosing is usually scaled to a patient's body size, measured either by body weight (kg) or by body surface area (BSA; measured in $m^2$, measured, or estimated by a combination of a patient's height and weight). Although there is no exact conversion between weight and BSA dosing, there is a good approximation: for a person of average weight and height (50th percentile for each), 25000 IU/kg = 1 MIU/$m^2$.

Treatment can be a single dose schedule or a multiple dose schedule. Doses can be given by bolus or by infusion. A typical treatment regimen for IL-2 is to administer 18 x $10^6$ IU per $m^2$ per 24-hours as a continuous infusion for 5 days, followed by 2-6 days without receiving IL-2, then an additional 5 days of intravenous IL-2 as a continuous infusion as before, and then 3 weeks without receiving IL-2. This is a single 'induction cycle' for IL-2. After the 3-week rest period of the first cycle, a second induction cycle can begin. Up to four maintenance cycles may be given with 4-week intervals to patients who respond or have disease stabilisation. If a patient cannot tolerate this dosage regimen, however, the dose should be reduced or administration interrupted until toxicity has moderated. Another treatment regimen for IL-2 is to administer 6 x $10^5$ IU per kg by intravenous bolus every 8 hours, for 14 doses over 5 days, with a second treatment being given after a 9 day rest period.

Preferred administrations include: intravenous infusion for treatment of metastatic renal cell carcinoma; intravenous infusion for treatment of metastatic melanoma; subcutaneous bolus for treatment of metastatic renal cell carcinoma; subcutaneous infusion for treatment of metastatic renal cell carcinoma; pulmonary administration by inhalation for treatment of metastatic renal cell carcinoma with lung metastasis.

***Combination therapies***

IL-2 microaggregates of the invention can be used as the active ingredient of pharmaceuticals. Such pharmaceuticals can be used on their own to treat patients, or can be used in conjunction with other active ingredients. Typically, the IL-2 will not be mixed with the other active ingredient before administration; rather, the IL-2 and the other active ingredient will be administered as separate independent medicines in a combined protocol. Combination therapy is particularly suited to treating cancers, including malignant and metastatic cancers.

Thus the invention provides (a) IL-2 microaggregates of the invention, and (b) a second pharmaceutical agent, for simultaneous separate or sequential administration.

The invention also provides a pharmaceutical preparation or system, comprising (a) a first pharmaceutical agent, which comprises IL-2 microaggregates of the invention; and (b) a second pharmaceutical agent, wherein said first and second agents are either in admixture or are separate compositions *e.g.* for simultaneous separate or sequential administration.

The invention also provides a kit comprising (a) a first pharmaceutical agent, which comprises IL-2 microaggregates of the invention; and (b) a second pharmaceutical agent.

The invention also provides the use of (a) IL-2 microaggregates of the invention and (b) a second pharmaceutical agent, in the manufacture of a combination medicament.

The invention also provides the use of IL-2 microaggregates of the invention in the manufacture of a medicament, wherein the medicament is for administration to a patient who has been pre-treated with a second pharmaceutical agent. Similarly, the invention provides the use of a second pharmaceutical agent in the manufacture of a medicament, wherein the medicament is for administration to a patient who has been pre-treated with IL-2 microaggregates of the invention. The pre-treatment may be recent (*e.g.* within the 24 hours preceding administration of said medicament), intermediate (*e.g.* more than 24 hours previous, but no longer than 4 weeks), more distant (*e.g.* at least 4 weeks previous), or very distant (*e.g.* at least 6 months previous), with these time periods referring to the most recent pre-treatment dose. The patient may be refractory to treatment by the pharmaceutical agent that was administered in the pre-treatment.

The invention also provides the use of IL-2 microaggregates of the invention in the manufacture of a medicament, wherein the medicament is co-administered with a second pharmaceutical agent. Similarly, the invention provides the use of a second pharmaceutical agent in the manufacture of a medicament, wherein the medicament is co-administered with IL-2 microaggregates of the invention. The two agents are preferably administered within 4 hours of each other.

Preferred second pharmaceutical agents are selected from the following table, which also includes details of the conditions which the combination therapy can be used to treat (and, where appropriate, basic details of how the combination therapy can be administered):

| Second agent | For treatment of |
|---|---|
| An anti-CD20 antibody, such as rituximab, ibritumomab or tositumomab *e.g.* Rituxan™, Mabthera™, Zevalin™, HuMax-CD20™, Bexxar™ | Non-Hodgkins lymphoma. Chronic Lymphocyte Leukemia.<br>Particularly where a NHL patient is refractory to rituximab treatment. |

(continued)

| Second agent | For treatment of |
|---|---|
| An anti-CD40 antibody. | Non-Hodgkins lymphoma. Multiple myeloma. Chronic Lymphocyte Leukemia. |
| An anti-Her2 antibody, such as trastuzumab *e.g.* Herceptin™ | Breast cancer.<br>Particularly where patient is refractory to trastuzumab treatment |
| An anti-EGFR antibody, such as cetuximab *e.g.* Erbitux™ | Non small cell lung cancer, or colorectal cancer. Particularly where a tumour over-expresses EGFR.<br>Particularly where a patient is refractory to cetuximab treatment. |
| An anti-VEGF antibody, such as bevacizumab *e.g.* Avastin™ | Colorectal cancer. |
| An anti-CD52 antibody, such as alemtuzumab *e.g.* Campath™ | Chronic Lymphocyte Leukemia. |
| An anti-CD33 antibody, such as gemtuzumab *e.g.* Mylotarg™ | Acute myeloid leukaemia. |
| A H2 receptor agonist, such as histamine or a pharmaceutically acceptable salt of histamine *e.g.* histamine dihydrochloride | Advanced malignant melanoma (*e.g.* at stage IV), acute myelogenous leukemia or renal cell carcinoma.<br>Particularly if the patient has liver metastasis.<br>Both agents can be given by subcutaneous injection.<br>For melanoma: IL-2 subcutaneous twice daily (BID) on days 1 & 2 of weeks 1 & 3, and days 1-5 of weeks 2 & 4; histamine diHCl subcutaneous over 10-30 mins on days 1-5 of all 4 weeks.<br>For acute myelogenous leukemia, commence after autologous stem cell transplantation or after chemotherapy. |
| Bacille Calmette-Guerin (BCG) | Bladder cancer.<br>IL-2 can be administered via Foley catheter. |
| Thalidomide | Non-Hodgkins lymphoma. |
| Lenalidomide | Multiple myeloma. |
| Proteasome inhibitors, such as bortezomib *e.g.* Velcade™ | Non-Hodgkins lymphoma. |
| 'CVP', which is a combination of cyclophosphamide, vincristine and prednisone. | Non-Hodgkins lymphoma.<br>Given by intravenous injection. |
| 'CHOP', which is combination of : cyclophosphamide, hydroxydoxorubicin, vincristine and prednisone. | Non-Hodgkins lymphoma.<br>Cyclophosphamide, hydroxydoxorubicin, vincristine are given by intravenous injection; prednisone is given by oral tablet. |
| 'CHOP-R', which is a combination of 'CHOP' with a rituximab. | Non-Hodgkins lymphoma.<br>Particularly for patients refractive to 'CHOP' or 'R' alone. |
| 'CVP-R', which is a combination of 'CVP' with a rituximab. | Non-Hodgkins lymphoma.<br>Particularly for patients refractive to 'CVP' or 'R' alone. |
| Interferon-$\alpha$ ('IFN$\alpha$') | Breast cancer, colorectal cancer or renal cell carcinoma. |
| Interferon-$\gamma$ ('IFN$\gamma$') | Cancer. |
| 5-fluorouracil ('5-FU') | Breast cancer, colorectal cancer or renal cell carcinoma. |

(continued)

| Second agent | For treatment of |
|---|---|
| Combination of 5-FU & IFNα | Breast cancer, colorectal cancer or renal cell carcinoma. |
| Anti-retroviral compounds | HIV. |
| A tyrosine kinase inhibitor, such as Bay43-9006 or SU11248 | Renal cell cancer or melanoma. |
| Decitabine | Thyroid cancer, myelodysplastic syndromes, melanoma or renal cell carcinoma. |

Where the second agent is an antibody, it is preferably a monoclonal antibody, more preferably a humanised or human antibody. Antibodies will generally be administered by injection *e.g.* subcutaneous or intravenous. Antibodies may be conjugated to other active agents *e.g.* tiuxetan, ozogamicin, radionuclides, $^{131}I$, *etc.*, particularly for cancer treatment.

In general, the second agent can be any antibody that provides ADCC (antibody-dependent cellular cytotoxicity). IL-2-based combination therapy is more useful with antibodies that mediate ADCC to treat cancer than with agents that treat cancer by inhibition of vascularisation or by altering cell cycle signal transduction.

***Definitions***

The term "comprising" encompasses "including" as well as "consisting" *e.g.* a composition "comprising" X may consist exclusively of X or may include something additional *e.g.* X + Y.

The term "about" in relation to a numerical value x means, for example, $x\pm10\%$. Where necessary, the term "about" can be omitted.

The word "substantially" does not exclude "completely" *e.g.* a composition which is "substantially free" from Y may be completely free from Y. Where necessary, the word "substantially" may be omitted from the definition of the invention.

Percent sequence identity can be determined using the Smith-Waterman homology search algorithm using an affine gap search with a gap open penalty of 12 and a gap extension penalty of 2, BLOSUM matrix of 62. The Smith-Waterman homology search algorithm is taught in reference 64.

**BRIEF DESCRIPTION OF THE DRAWINGS**

Figure 1 shows the increase in specific turbidity (τ) as SDS is removed during diafiltration.

Figures 2 and 3 show the amino acid sequence of IL-2 in the PROLEUKIN™ product (SEQ ID NO: 1). This sequence (i) lacks the N-terminus methionine of SEQ ID NO: 3, (ii) lacks the alanine residue found at the N-terminus of natural mature human IL-2 as seen in SEQ ID NO: 4, and (iii) has a serine substitution at the Cys-125 residue seen in SEQ ID NO: 4.

Figure 4 shows a nucleotide sequence encoding the Figure 2 sequence (SEQ ID NO: 2, which includes the start and stop codons, and is expressed as SEQ ID NO: 3 before cleavage of N-terminus methionine). Numbers at the left indicate the number of the first base in each row, as counted from the 5' end of the (+) DNA strand of the IL-2 gene. Numbers under the amino acid sequence indicate residue numbers, as counted from the N-terminus of native human IL-2. Arrows indicate nucleotide differences relative to human IL-2 (*i.e.* the differences between SEQ ID NOS: 2 and 5).

Figure 5 shows the specific turbidity (τ, $cm^2/g$) of seven samples used for pharmacokinetic testing, with different SDS concentrations (μg SDS per mg IL-2), and Figure 6 shows the proportion of total IL-2 which could be pelleted by centrifugation in the seven samples.

Figure 7 shows plasma bioactivity for six of the seven samples. Figure 8 compares the 160μg/mg and 25μg/mg samples, and Figure 9 is a composite of Figures 7 and 8. X axes show time (hours) after injection; Y axes show plasma bioactivity (ng/ml).

Figure 10 shows τ ($cm^2$/g) against SDS concentration (mg/μg) during diafiltration, and Figure 11 converts τ into MW (kDa).

Figure 12 shows the drop in SDS concentration during diafiltration.

Figure 13 superimposes the turbidity results of Figure 10 on the SDS removal profile of Figure 12.

Figure 14 shows the effect of lyophilisation on τ ($cm^2$/g). Squares show pre-lyophilisation values, and circles show post-lyophilisation values. X axis shows SDS concentration of the samples (μg/mg).

Figure 15 shows the bioactivity of IL-2 microaggregates, before and after dilution with serum, and a comparison with monomeric IL-2.

Figures 16 and 17 show dynamic light scattering spectra for IL-2 microaggregates. Figure 16 is distributed by mass of the solution's components, and Figure 17 is distributed by number.

## MODES FOR CARRYING OUT THE INVENTION

### *1/ Preparation of IL-2 microaggregates*

*E.coli* transformed with a plasmid harbouring the Figure 4 nucleotide sequence were cultured under standard conditions, such that IL-2 protein having the sequence of Figure 2 was expressed. After culture, IL-2 was predominantly in the form of inclusion bodies.

Primary recovery of IL-2 included concentration, cell disruption, diafiltration, inactivation, redisruption and sucrose suspension. Concentration of the harvested cells was performed using crossflow filters in order to decrease the working volume approximately five-fold. The cells were disrupted by homogenization to facilitate isolation of the IL-2 inclusion bodies. Fermentation media salts were removed by diafiltration and the host bacteria in the diafiltered solution were killed by the addition of an alcohol. After alcohol treatment, the cells were disrupted by homogenization. Sucrose was added to obtain a mixture of higher density, in order to permit separation of the IL-2 inclusion bodies from the cell debris during centrifugation. The IL-2 particle paste was collected by centrifugation, aliquoted and stored at -70°C or colder, until further processing.

The IL-2 particle paste was solubilised with SDS and reduced with dithiothreitol (DTT) in order to obtain a monomeric form of soluble IL-2 without any disulfide bond. This process step was performed to increase yield when the IL-2 is chromatographed in the next step.

The monomeric soluble form of IL-2 was purified by a process including the steps of size exclusion chromatography (SEC), oxidation, concentration, RP-HPLC, precipitation and centrifugation. The first chromatographic step of SEC was performed to separate IL-2 from host proteins and nucleic acids. The fractions containing IL-2 were collected and pooled. The IL-2 was oxidized in order to obtain the intramolecular disulfide bond. Concentration of the IL-2 solution was performed using ultrafiltration in order to decrease the working volume. The second chromatographic step, preparative RP-HPLC, was performed to separate IL-2 from bacterial endotoxins, nucleic acids, and host proteins, and to remove IL-2 isoforms, including oxidized forms. The fractions containing IL-2 were collected and pooled. The IL-2 was precipitated by addition of sodium hydroxide (NaOH) and collected by centrifugation. This process step was performed to remove solvents used in the RP-HPLC purification. The material at this stage is referred to as the 'HPLC paste'.

The HPLC paste was then resolubilised by adding a solution of 0.05 M $Na_2HPO_4$ + 1% SDS. When SDS is added to a sufficient concentration then the composition contains mainly monomeric solubilised IL-2, but a small amount of oligomerised IL-2 (*i.e.* covalently-linked IL-2 molecules in the form of oligomers) may have formed during purification. These oligomers were removed by gel filtration, which also removes any residual organic solvents from previous steps.

As SDS can be toxic to cells, it is removed from the solubilised IL-2. The solution was diafiltered against 10 mM sodium phosphate buffer, pH 7.5, until the SDS level reaches approximately 160μg/mg IL-2. Microaggregates of IL-2 and SDS begin to form at about 500-600μg/mg, and their size gradually increases as the SDS level is further decreased to the target 160μg/mg level (Figure 1). This SDS removal, which is key to the formation of microaggregates, is the last step in the purification process of IL-2, prior to formulation.

### *2/ Formulation of IL-2 microaggregates for pharmaceutical use*

The diafiltered SDS/IL-2 mixture, containing microaggregates, was tested for protein concentration (mg/ml) by dividing its absorbance at 280 nm by the IL-2 extinction coefficient (0.79). The volume of the diafiltered IL-2 solution was also measured (ml). The total amount of IL-2 protein (mg) was calculated by multiplying the volume of the diafiltered IL-2 solution by the protein concentration. The final working volume (ml) for formulation was calculated by multiplying the total mg of protein by the factor 0.909. This factor takes into account IL-2 loss during filtration.

To stabilise the IL-2 during lyophilisation, a ¼ volume of a 20% mannitol solution was added, to give a final mannitol concentration of 5% (*i.e.* 50mg/ml). A 10 mM sodium phosphate buffer pH 7.5 was also added up to the final working volume. After the mannitol and buffer were added, the IL-2 concentration in the formulated solution was determined by measuring the absorbance at 280nm.

The end result after formulation was a bulk solution at pH 7.5, containing: IL-2 at a final concentration of 1.1±0.05mg/ml; 0.18mg/ml SDS; 50mg/ml mannitol; 0.17mg/ml monobasic sodium phosphate; and 0.89mg/ml dibasic sodium phosphate.

The formulated IL-2 bulk solution was filter sterilised into a sterile bulk tank. Material is transferred from this tank into sterile vials. 1.2ml of bulk solution was introduced into each vial. The vials were then placed in a lyophilizer and freeze-dried. Stoppers were then applied, and the stoppers were over-sealed. The vials were then labelled and packaged.

***3/ Effect of SDS concentration on turbidity***

Before arriving at the preferred concentration range of SDS, IL-2 was prepared by the method described above, except that large samples (~300mg IL-2) were removed at various points during the diafiltration for turbidity investigation, in order to establish the effect of SDS on IL-2 aggregation.

Figure 12 shows the SDS concentration during diafiltration of the starting material. The concentration decreases as a straight line on the logarithmic axis. Samples were removed at the following SDS concentrations (μg per mg IL-2): 1195, 536, 351, 217, 173, 137, 123, 104 and 72.

The IL-2 purity was the same in each sample (100% by SDS-PAGE, ≥98.5% by HPLC). Turbidity was measured at 488nm and converted to an absolute specific turbidity (τ) as described above, based on IL-2 concentration in the samples. Results are shown in Figure 10, and Figure 13 superimposes the turbidity results on the SDS removal profile of Figure 12.

Below about 300μg/mg the turbidity gradually increases, and the increase becomes very steep below about 95μg/mg.

The τ values can be converted to inferred molecular weights by the following formula:

$$MW = \frac{\tau \times 10^7}{8.1 + 2\tau}$$

The results of this conversion for pre-lyophilisation samples are shown in Figure 11. These and other data show that a MW corresponding to monomeric IL-2 is seen above about 600μg/mg, and MW starts to increase significantly when SDS is below about 300μg/mg, with the increase becoming very steep when SDS drops below about 100μg/mg.

Based on the MW of monomeric IL-2 (15.3kDa for native IL-2), the molecular weight of an aggregate can be converted into an inferred number of IL-2 monomers per aggregate.

***4/ Lyophilisation and specific turbidity***

During diafiltration for SDS removal, 15ml samples were removed at the following SDS concentrations: 1155; 840; 585; 400; 190; and 155 μg/mg IL-2. Turbidity of these samples was determined both before and after formulation (*i.e.* after addition of mannitol, phosphate buffer, lyophilisation and reconstitution). In both cases, turbidity measurements were preceded by filtration through a 0.2μm filter in order to remove any large aggregates.

Figure 14 shows the effect of lyophilisation on τ (logarithmic scale) for these samples. The graph shows that lyophilisation does not destroy the microaggregates, but that it can often increase specific turbidity slightly (*i.e.* the aggregates increase in size), without affecting the dependence on SDS concentration. The effect is generally minor over the preferred SDS range.

***5/ Dynamic light scattering***

IL-2 microaggregates were prepared as described above, with a SDS concentration of 160μg/mg, and were analysed by DLS. The results are shown in Figures 16 and 17. Figure 16 shows the DLS results distributed by mass of the solution's components, and Figure 17 shows them by number.

In Figure 16, the main peak has a mean hydrodynamic diameter of 12.7nm, representing 99.02% of the total mass. There is also a downstream peak with a mean diameter of 55.6nm, representing the remaining 0.98% of mass. In Figure 17, the main peak has a mean hydrodynamic diameter of 11.7nm, representing 99.99% of the total number. There is also a downstream peak with a mean diameter of 53.7nm, representing the remaining 0.01% of particles.

### *6/ Comparison of microaggregates* **vs.** *monomeric IL-2*

Microaggregated IL-2 was compared to monomeric IL-2 in various tests. IL-2 microaggregates were prepared as described above. For comparative purposes, IL-2 was prepared in a monomeric form, in which Tween 80 was used as the detergent instead of SDS. This detergent does not form microaggregates with IL-2, but the detergent does solubilised the IL-2 and gives a bioactive monomeric product.

Both forms of IL-2 were confirmed to be bioactive. The aggregated IL-2 had an activity of around 20MIU/mg. When diluted with serum *ex vivo,* activity dropped slightly. In both cases, activity was slightly higher than that of monomeric IL-2 (Figure 15). Thus IL-2 microaggregates of the invention are able to dissociate to give a bioactive product, and bioactivity is equivalent to that of IL-2 that has not been aggregated.

The *in vivo* distribution of radiolabelled IL-2 was compared after intravenous administration of the protein either in SDS-microaggregated form or in monomeric form. The microaggregated form preferentially distributed to lung, liver and kidney, whereas the monomeric product distributed almost 100% to the kidney. Microaggregation therefore has an impact on *in vivo* distribution.

Microaggregated and monomeric IL-2 were also compared in a tumour metastasis model. B16F10 tumour cells were intravenously injected into the tails of mice and, 14 days later, lungs were inspected for tumours. Treated animals received IL-2 daily from days 3-10. The median numbers of lung metastases at day 14 were as follows:

| Dose (mg/kg) | IL-2/SDS microaggregates | IL-2/Tween 80 monomeric |
|---|---|---|
| 15 | 1 | 16 |
| 10 | 3 | 26 |
| 7.5 | 4.5 | 49 |
| 5 | 24 | 53.5 |
| 1 | 60 | 59.5 |
| 0.1 | 88.5 | 72 |

The microaggregated form of IL-2 is thus more effective in the tumour model than the monomeric form, in that metastases can be prevented using much lower doses of IL-2. Because IL-2 can itself be toxic, however, the data are not directly comparable *e.g.* 10mg/kg IL-2 microaggregates can kill about ½ a test population. The most meaningful comparison is thus at the highest non-lethal dose, namely at the 7.5mg/kg dose. At this equitoxic dose, the median number of metastases seen with microaggregates was 4.5 (range 1-18), compared to 16 (range 4-57) with monomeric IL-2, with the difference being statistically significant. This difference represents a 3- to 5-fold better therapeutic index for the microaggregated form of IL-2.

Further studies with this B16 artificial metastasis model revealed that the SDS levels in the final product do not appear to impact efficacy. For instance, a composition with 160μg/mg of SDS performed in the same way as one with 181μg/mg. With SDS in the range 95-250μg/mg, therefore, anti-tumour activity remains optimal while SDS-related toxicity is avoided.

### *7/ Pharmacokinetic effects of final SDS concentration*

As shown above, turbidity of IL-2/SDS mixtures increases as SDS levels decrease during diafiltration. In a pharmacokinetic test, the diafiltration was continued well beyond the typical target of 160μg/mg, right down to 25μg/mg. Samples were taken during the diafiltration process at various stages: 1090μg/mg; 200μg/mg; 160μg/mg; 125μg/mg; 95μg/mg; 65μg/mg; and 25μg/mg.

All samples except the most diluted (25μg/mg) were visibly clear, and Figure 5 shows the specific turbidity ($\tau$) of the seven samples. The drop in $\tau$ at the lowest concentration reflects a huge increase in protein precipitation at 25μg/mg, which was visible to the eye. Figure 6 shows the proportion of total IL-2 which could be pelleted by centrifugation in the seven samples, and a large increase is seen at the lowest SDS concentration whereas the higher concentrations are essentially constant. This precipitated material settled out and so did not cause turbidity, explaining the low $\tau$ value in Figure 5.

In contrast to the varying turbidity and precipitation, bioactivity (IU) was essentially constant for the seven samples (around 19 IU/mg).

To study pharmacokinetics, all samples except the most diluted were injected as a bolus into the talk vein of rats (triplicate per SDS dose, 18 rats in total) at 0.2mg IL-2 per kg body weight, based on an allometric extrapolation of a human dose. Blood samples were taken at 1, 3, 5, 7.5, 10, 15, 20, 25, 30, 45 and 60 minutes after injection and were assayed for IL-2 bioactivity. Figure 7 shows plasma bioactivity (ng/ml) of these samples. Between 0.5 hours and I hour, the top line is the 160μg/mg dose (■), and the lowest line is 65μg/mg (Δ), although all lines are very similar.

In a second study, the sample with the highest line from the first experiment (160μg/mg) was compared to the most diluted sample (25μg/mg). The pharmacokinetic parameters of the 160μg/mg sample in the second study matched those from the first study. Whereas the lines in Figure 7 are all similar shapes to each other, however, the most diluted sample gave very different results (Figure 8). The clearance rate calculated from the curves were ~30-fold different: 12.7ml/min/kg for the 160μg/mg dose, and 362ml/min/kg for the 25μg/mg dose.

When the results from the two experiments are compared, the most diluted sample is clearly distinct from the other six samples (Figure 9).

Thus the aggregation status of IL-2 has a clear effect on its *in vivo* pharmacokinetics.

***8/ Free SDS***

The above experiments measure the total SDS concentration in SDS/fL-2 mixtures. Experiments were performed to see how much of the total SDS was associated with IL-2 and how much remains free in solution.

Vials of PROLEUKIN™ product were reconstituted as directed and were ultrafiltered through Centricon 10 membranes (10kDa cutoff) by two centrifugations at 3000rpm for 30 minutes each. The starting sample and the filtrate were tested for SDS and IL-2 levels. SDS was measured by the acridine orange assay [35]. Briefly, this assay involves: take 0.5ml of sample; add 0.1ml of 1.75M $NaHSO_4$ solution; add 0.1ml of 1% (w/v) acridine orange solution; add 1.5ml toluene; seal; vortex for 3 minutes; separate organic and aqueous phases *e.g.* by centrifugation for 5 minutes at 3000rpm and ambient temperature; measure absorbance of the upper organic layer at 499nm. Samples may be diluted (*e.g.* with pure water) prior to the assay to bring them within the range of a standard curve *e.g.* if the standard curve is based on known SDS concentrations up to 25μg/ml then the sample should be diluted to bring its SDS concentration into this range. The blank for the spectrophotometric step should be a standard containing zero SDS that has been treated in the same way as the experimental sample.

Results showed that IL-2 was totally retained by the ultrafiltration membranes (none detectable in the filtrate), whereas about 35% of SDS was able to pass through the membrane.

**REFERENCES** (the full contents of which are incorporated herein by reference)

[1] Hora et al. (1990) Biotechnology (N Y) 8:755-8.

[2] US patent 4,569,790.

[3] Meyers et al. (1991) Clin Pharmacol Ther. 49:307-13.

[4] US patent 4,604,377.

[5] Hora et al. (1992) Dev Biol Stand 74:295-306.

[6] WO85/04328.

[7] US patent 4,748,234.

[8] Ho et al. (1992) Vaccine 10:209-13.

[9] Geigert et al. (1993) Chapter 8 of Stability and Characterization of Protein and Peptide Drugs: Case Histories (eds. Wang & Pearlman). ISBN 0-306-44365-1.

[10] Vemuri (1992) Dev Biol Stand. 74:341-51.

[11] Anderson et al. (1992) Am J Hosp Pharm 49:608-12.

[12] Johnston et al. (1992) Pharm Res 9:425-34.

[13] Anderson et al. (1992) J Immunother 12:19-31.

[14] Anderson & Sorenson (1994) Clin Pharmacokinet 27:19-31.

[15] Konigsberg et al. (1998) Biochim Biophys Acta 1370:243-51.

[16] Kanaoka et al. (2001) J Pharm Pharmacol 53:295-302.

[17] Ozbas-Turan et al. (2002) J Pharm Sci 91:1245-51.

[18] Bergmann et al. (1991) Mol Immunol 28(1-2):99-105..

[19] US patent 4,908,434.

[20] US patent 4,925,919.

[21] US patent 4,853,332.

[22] US patent 5,464,939.

[23] US patent 5,824,330.

[24] US patent 4,778,879.

[25] US patent 4,992,271.

[26] US patent 5,037,644.

[27] US patent 5,078,997.

[28] US patent 4,816,440.

[29] EP-B-0268110.

[30] EP-B-0217645.

[31] Heinig & Vogt (1999) Electrophoresis 20:3311-28.

[32] Nomura & Murakami (1999) Bunseki Kagaku 48:111-6.

[33] Fielden & Claesson (1998) J Colloid Interface Sci 198:261-5.

[34] Gabor & May (1996) Abstract # C-04, 1996 FDA Science Forum Poster Abstracts.

[35] Sokoloff & Frigon (1981) Analytical Biochem 118:138-41.

[36] Arakawa et al. (1994) Int J Pept Protein Res 43:583-7

[37] US patent 4,738,927.

[38] US patent 4,656,132.

[39] US patent 4,530,787.

[40] US patent 4,572,798.

[41] US patent 4,931,543.

[42] Chapter 8 of Biological Spectroscopy (Campbell & Dwek). ISBN 0-8053-1849-6.

[43] Piccora (1985) Dynamic Light Scattering. Plenum Press, New York.

[44] US patent 4,518,584.

[45] Bazan (1992) Science 257:410-2.

[46] Wang et al. (1984) Science 224:1431-3.

[47] McKay (1992) Science 257:412.

[48] Theze et al. (1996) Immunol Today 17:481-6.

[49] Buchli & Ciardelli (1993) Arch Biochem Biophys 307:411-5.

[50] Collins et al. (1988) PNAS USA 85:7709-13.

[51] Kuziel et al. (1993) J Immunol 150:5731.

[52] Eckenberg et al. (1997) Cytokine 9:488-98.

[53] EP-A-0136489.

[54] US patent 4,752,585.

[55] EP-A-0200280.

[56] WO99/60128.

[57] US patent 5,229,109.

[58] WO00/58456.

[59] US patent 6,168,785.

[60] Ahmad et al. (1994) J Protein Chem 13:591-8.

[61] Gearing & Thorpe (1988) J Immunol Methods 114:3-9.

[62] Rossio (1997) Cytokines and immune cell products. Pages 348-356 of Manual of Clinical and Laboratory Immunology, 5th edition (eds. Rose, deMacario, Folds, Lane, Nakamura).

[63] Wadhwa et al. (2000) Quantitative biological assays for individual cytokines. Pages 207-239 of Cytokine Cell Biology: A Practical Approach (ed. Balkwill). ISBN 0-19-963860-8.

[64] Smith & Waterman (1981) Adv. Appl. Math. 2:482-9.

## Claims

1. A process for preparing interleukin-2 for pharmaceutical use, comprising the steps of: (a) adding sodium dodecyl sulfate (SDS) to a composition comprising IL-2 with intramolecular disulfide bridges, to give a final SDS concentration of at least 500μg SDS per mg of IL-2; (b) removing any precipitated covalently-linked IL-2 multimers by gel filtration; and (c) reducing the SDS concentration to remove some but not all SDS from the composition, to give a composition that contains between 95-250μg SDS per mg of IL-2.

2. The process of claim 1, comprising the steps of: purifying IL-2 by RP-HPLC; precipitating the IL-2; adding SDS to the precipitated IL-2, to give a final SDS concentration of at least 500μg SDS per mg of IL-2; removing any precipitated IL-2 covalent multimers by gel filtration; and reducing the SDS concentration to remove some but not all SDS from the composition, to give a composition that contains between 95-250μg SDS per mg of IL-2.

3. The process of any preceding claim, wherein the final SDS concentration in step (a) is at least 900 μg/mg.

4. The process of any preceding claim, wherein the product of step (c) has a SDS concentration between 135-180 μg/mg.

5. A process for preparing a composition comprising interleukin-2 and sodium dodecyl sulfate, wherein: (i) interleukin-2 and sodium dodecyl sulfate in the composition are present in the form of aggregates; and (ii) the process comprises the steps of (a) adding sodium dodecyl sulfate to a composition comprising interleukin-2 with intramolecular disulfide bridges, wherein sufficient sodium dodecyl sulfate is added to give monomeric solubilised interleukin-2, (b) removing any precipitated covalently-linked IL-2 multimers by gel filtration, and (c) reducing the concentration of sodium dodecyl sulfate to a level where the interleukin-2 forms aggregates with sodium dodecyl sulfate, such that the composition, after lyophilisation and reconstitution, has a specific turbidity (τ) of less than 1.1 $cm^2/g$.

6. The process of claim 5, wherein the aggregates have a mean hydrodynamic diameter of between 8 nm and 20 nm.

7. The process of any preceding claim, wherein the SDS concentration is reduced by diafiltration.

8. The process of any preceding claim, further comprising a step of: addition of mannitol.

9. The process of any preceding claim, further comprising a step of: lyophilisation.

10. A composition comprising interleukin-2 and sodium dodecyl sulfate, wherein IL-2 and sodium dodecyl sulfate are present in the form of aggregates, and wherein the composition has one or more of the following characteristics:

    (i) the composition has a specific turbidity (τ) less than 1.1 $cm^2/g$;
    (ii) the composition contains SDS/IL-2 aggregates with a mean hydrodynamic diameter of between 8 nm and 20 nm;
    (iii) the composition contains between 95-250μg SDS per mg of IL-2; and/or
    (iv) the aggregates are obtainable by the process of any preceding claim.

**11.** A lyophilised composition comprising interleukin-2 and sodium dodecyl sulfate, wherein the composition can be reconstituted with an aqueous medium to give the composition of claim 10.

**12.** The composition of claim 10, wherein the specific turbidity of the composition is less than 0.7 $cm^2/g$.

**13.** The composition of claim 12, wherein the specific turbidity of the composition is between 0.3 $cm^2/g$ and 0.6 $cm^2/g$.

**14.** The composition of any one of claims 10 to 13, wherein the composition comprises SDS/IL-2 microaggregates with a hydrodynamic diameter between 11 nm and 13 nm.

**15.** The composition of any one of claims 10 to 14, wherein the composition includes mannitol at a concentration between 40 and 50mg mannitol per mg IL-2.

**16.** The composition of any one of claims 10 to 15, wherein the IL-2 is a human IL-2.

**17.** The composition of any one of claims 10 to 16, wherein the IL-2 is a des-alanyl-1 IL-2.

**18.** The composition of any one of claims 10 to 17, wherein the IL-2 is a serine-125 IL-2.

**19.** The composition of any one of claims 10 to 18, wherein the IL-2 is des-alanyl-1, serine-125 human IL-2.

**20.** The composition of any one of claims 10 to 19, wherein the IL-2 has the amino acid sequence SEQ ID NO: 1.

**21.** The composition of any one of claims 10 to 20, wherein the IL-2 is non-glycosylated.

**22.** The composition of any one of claims 10 to 21, wherein the IL-2 is purified after expression in a recombinant prokaryotic host.

**23.** The composition of any one of claims 10 to 22, with an IL-2 concentration of about 1.1 mg/ml.

**24.** The lyophilised composition of claim 11 which, when reconstituted with 1.2 ml sterile water for injection, contains in each millilitre: 1.1 mg non-glycosylated des-atanyl-1, serine-125 human IL-2; 50 mg mannitol; 0.18 mg sodium dodecyl sulfate (SDS); 0.17 mg monobasic sodium phosphate; and 0.89 mg dibasic sodium phosphate to pH 7.5, wherein the IL-2 and SDS are in the form of aggregates such that the reconstituted composition has a specific turbidity ($\tau$) less than 1.1 $cm^2/g$.

**25.** An aqueous pharmaceutical composition, obtainable by reconstituting the lyophilised composition of claim 24 with 1.2 ml sterile water for injection.

**26.** The composition of any one of claims 10 to 25, and a second pharmaceutical agent, for simultaneous separate or sequential administration.

**27.** A kit comprising (a) the composition of any one of claims 10 to 25; and (b) a second pharmaceutical agent.

**28.** The composition of any one of claims 10 to 25, for use in medicine.

**29.** The use of a composition comprising both interleukin-2 and sodium dodecyl sulfate, in the manufacture of a medicament for administration to a patient, wherein the IL-2 and sodium dodecyl sulfate are present in the form of aggregates, and wherein the composition has one or more of the following characteristics:

(i) the composition has a specific turbidity ($\tau$) less than 1.1 $cm^2/g$;
(ii) the composition contains SDS/IL-2 aggregates with a mean hydrodynamic diameter of between 8 nm and 20 nm;
(iii) the composition contains between 95-250$\mu$g SDS per mg of IL-2; and/or
(iv) the aggregates are obtainable by the process of any one of claims 1 to 9.

**30.** The use of claim 29, wherein the medicament is for treating cancer.

31. The use of claim 30, wherein the cancer is renal cell carcinoma.

32. The use of claim 31, wherein the cancer is melanoma.

33. The use of any one of claims 29 to 32, wherein the patient has been pre-treated with a second pharmaceutical agent.

34. The use of any one of claims 29 to 32, wherein the medicament is co-administered with a second pharmaceutical agent.

35. The use of a second pharmaceutical agent, in the manufacture of a combination medicament for administration to a patient, wherein the patient has been pre-treated with the composition of any one of claims 10 to 25.

36. The use of a second pharmaceutical agent, in the manufacture of a combination medicament for administration to a patient, wherein the medicament is co-administered with the composition of any one of claims 10 to 25.

37. The use of (a) microaggregates of interleukin-2 and sodium dodecyl sulfate as defined in claim 10, and (b) a second pharmaceutical agent, in the manufacture of a combination medicament, wherein the microaggregates have a mean hydrodynamic diameter of between 8 nm and 20 nm.

38. The composition of claim 26, wherein the second pharmaceutical agent is a monoclonal antibody.

39. The composition of claim 26, wherein the second pharmaceutical agent is an antibody that provides ADCC.

40. The composition of claim 26, wherein the second pharmaceutical agent is selected from the group consisting of: an anti-CD20 antibody; an anti-CD40 antibody; an anti-Her2 antibody; an anti-EGFR antibody; and anti-VEGF antibody; an anti-CD52 antibody; an anti-CD33 antibody; a H2 receptor agonist; Bacille Calmette-Guerin; thalidomide; Lenalidomide; a proteasome inhibitor; a combination of cyclophosphamide, vincristine and prednisone; a combination of cyclophosphamide, hydroxydoxorubicin, vincristine and prednisone; a cytokine; interferon $\alpha$; interferon $\gamma$; 5-fluorouracil; an anti-retroviral compound; a tyrosine kinase inhibitor; and Decitabine.

41. The composition of claim 40, wherein the H2 receptor agonist is histamine or a pharmaceutically acceptable salt of histamine, such as histamine dihydrochloride.

42. The use of any one of claims 33 to 37, wherein the second pharmaceutical agent is a monoclonal antibody.

43. The use of any one of claims 33 to 37, wherein the second pharmaceutical agent is an antibody that provides ADCC.

44. The use of any one of claims 33 to 37, wherein the second pharmaceutical agent is selected from the group consisting of: an anti-CD20 antibody; an anti-CD40 antibody; an anti-Her2 antibody; an anti-EGFR antibody; and anti-VEGF antibody; an anti-CD52 antibody; an anti-CD33 antibody; a H2 receptor agonist; Bacille Calmette-Guerin; thalidomide; Lenalidomide; a proteasome inhibitor; a combination of cyclophosphamide, vincristine and prednisone; a combination of cyclophosphamide, hydroxydoxorubicin, vincristine and prednisone; a cytokine; interferon $\alpha$; interferon $\gamma$; 5-fluorouracil; an anti-retroviral compound; a tyrosine kinase inhibitor; and Decitabine.

45. The use of claim 44, wherein the H2 receptor agonist is histamine or a pharmaceutically acceptable salt of histamine, such as histamine dihydrochloride.

**Patentansprüche**

1. Verfahren zur Aufbereitung von Interleukin-2 zur pharmazeutischen Verwendung, umfassend die Schritte: (a) Zugabe von Natriumdodecylsulfat (SDS) zu einer Zusammensetzung umfassend IL-2 mit intramolekularen Disulfidbrükken bis zu einer SDS-Endkonzentration von mindestens 500 $\mu$g SDS pro mg IL-2; (b) Entfernen jeglicher ausgefällter kovalent verknüpfter IL-2-Multimere durch Gelfiltration; und (c) Reduzieren der SDS-Konzentration, um SDS teilweise aber nicht vollständig aus der Zusammensetzung zu entfernen, um eine Zusammensetzung zu erhalten, welche zwischen 95-250 $\mu$g SDS pro mg IL-2 enthält.

2. Verfahren nach Anspruch 1, umfassend die Schritte: Reinigen von IL-2 durch RP-HPLC; Ausfällen des IL-2; Zugabe von SDS zum ausgefälltem IL-2 bis zu einer SDS-Endkonzentration von mindestens 500 μg SDS pro mg IL-2; Entfernen jeglicher ausgefällter kovalenter IL-2-Multimere durch Gelfiltration; und Reduzieren der SDS-Konzentration, um SDS teilweise aber nicht vollständig aus der Zusammensetzung zu entfernen, um eine Zusammensetzung zu erhalten, welche zwischen 95-250 μg SDS pro mg IL-2 enthält.

**3.** Verfahren nach einem der vorangehenden Ansprüche, wobei die SDS-Endkonzentration in Schritt (a) mindestens 900 μg/mg beträgt.

**4.** Verfahren nach einem der vorangehenden Ansprüche, wobei das Produkt aus Schritt (c) eine SDS-Konzentration zwischen 135-180 μg/mg hat.

**5.** Verfahren zur Herstellung einer Zusammensetzung umfassend Interleukin-2 und Natriumdodecylsulfat, wobei: (i) Interleukin-2 und Natriumdodecylsulfat in der Zusammensetzung in Form von Aggregaten vorliegen, und (ii) das Verfahren die Schritte umfasst: (a) Zugabe von Natriumdodecylsulfat zu einer Zusammensetzung umfassend Interleukin-2 mit intramolekularen Disulfidbrücken, wobei ausreichend Natriumdodecylsulfat zugegeben wird, um monomeres solubilisiertes Interleukin-2 zu erhalten, (b) Entfernen jeglicher ausgefällter kovalent-verknüpfter IL-2-Multimere durch Gelfiltration; und (c) Reduzieren der Natriumdodecylsulfat-Konzentration bis zu einem Spiegel, bei dem das Interleukin-2 Aggregate mit Natriumdodecylsulfat bildet, so dass die Zusammensetzung, nach Lyophilisation und Wiederherstellung, eine spezifische Trübung (τ) von weniger als 1,1 $cm^2/g$ hat.

**6.** Verfahren nach Anspruch 5, wobei die Aggregate einen mittleren hydrodynamischen Durchmesser zwischen 8 nm and 20 nm haben.

**7.** Verfahren nach einem der vorangehenden Ansprüche, wobei die SDS-Konzentration durch Diafiltration reduziert wird.

**8.** Verfahren nach einem der vorangehenden Ansprüche, weiterhin umfassend den Schritt: Zugabe von Mannit.

**9.** Verfahren nach einem der vorangehenden Ansprüche, weiterhin umfassend den Schritt: Lyophilisation.

**10.** Zusammensetzung, umfassend Interleukin-2 und Natriumdodecylsulfat, wobei IL-2 und Natriumdodecylsulfat in Form von Aggregaten vorliegen und wobei die Zusammensetzung eine oder mehrere der folgenden Eigenschaften hat:

(i) die Zusammensetzung hat eine spezifische Trübung (τ) von weniger als 1,1 $cm^2/g$;
(ii) die Zusammensetzung enthält SDS/IL-2-Aggregate mit einem mittleren hydrodynamischen Durchmesser zwischen 8 nm und 20 nm;
(iii) die Zusammensetzung enthält zwischen 95-250 μg SDS pro mg IL-2; und/oder
(iv) die Aggregate sind durch ein Verfahren nach einem der vorangehenden Ansprüche erhältlich.

**11.** Lyophilisierte Zusammensetzung, umfassend Interleukin-2 und Natriumdodecylsulfat, wobei die Zusammensetzung mit einen wässrigen Medium wiederhergestellt werden kann, um die Zusammensetzung nach Anspruch 10 zu erhalten.

**12.** Zusammensetzung nach Anspruch 10, wobei die spezifische Trübung der Zusammensetzung kleiner als 0,7 $cm^2/g$ ist.

**13.** Zusammensetzung nach Anspruch 12, wobei die spezifische Trübung der Zusammensetzung zwischen 0,3 $cm^2/g$ und 0,6 $cm^2/g$ liegt.

**14.** Zusammensetzung nach einem der Ansprüche 10 bis 13, wobei die Zusammensetzung SDS/IL-2-Mikroaggregate mit einem hydrodynamischen Durchmesser zwischen 11 nm und 13 nm umfasst.

**15.** Zusammensetzung nach einem der Ansprüche 10 bis 14, wobei die Zusammensetzung Mannit in einer Konzentration zwischen 40 und 50 mg Mannit pro mg IL-2 enthält.

**16.** Zusammensetzung nach einem der Ansprüche 10 bis 15, wobei das IL-2 menschliches IL-2 ist.

**17.** Zusammensetzung nach einem der Ansprüche 10 bis 16, wobei das IL-2 ein Des-Alanyl-1-IL-2 ist.

**18.** Zusammensetzung nach einem der Ansprüche 10 bis 17, wobei das IL-2 ein Serin-125-IL-2 ist.

**19.** Zusammensetzung nach einem der Ansprüche 10 bis 18, wobei das IL-2 menschliches Des-Alanyl-1-Serin-125-IL-2 ist.

**20.** Zusammensetzung nach einem der Ansprüche 10 bis 19, wobei das IL-2 die Aminosäuresequenz SEQ ID NO: 1 hat.

**21.** Zusammensetzung nach einem der Ansprüche 10 bis 20, wobei das IL-2 nichtglykosyliert ist.

**22.** Zusammensetzung nach einem der Ansprüche 10 bis 21, wobei das IL-2 nach Expression in einem rekombinaten prokaryontischen Wirt gereinigt wird.

**23.** Zusammensetzung nach einem der Ansprüche 10 bis 22 mit einer IL-2-Konzentration von etwa 1,1 mg/ml.

**24.** Lyophilisierte Zusammensetzung nach Anspruch 11, welche, wenn sie mit 1,2 ml sterilem Wasser zur Injektion wiederhergestellt wurde, in jedem Milliliter enthält: 1,1 mg nicht-glykosyliertes menschliches Des-Alanyl-1-Serin-125-IL-2; 50 mg Mannit; 0,18 mg Natriumdodecylsulfat (SDS); 0,17 mg monobasisches Natriumphosphat; und 0,89 mg dibasisches Natriumphosphat zu pH 7,5, wobei IL-2 und SDS in Form von Aggregaten vorliegen, so dass die wiederhergestellte Zusammensetzung eine spezifische Trübung ($\tau$) von weniger als 1,1 $cm^2/g$ hat.

**25.** Wässriges Arzneimittel, erhältlich durch Wiederherstellen der lyophilisierten Zusammensetzung nach Anspruch 24 mit 1,2 ml sterilem Wasser zur Injektion.

**26.** Zusammensetzung nach einem der Ansprüche 10 bis 25, und ein zweites Arzneimittel für simultane, separate oder sequenzielle Verabreichung.

**27.** Kit, umfassend (a) die Zusammensetzung nach einem der Ansprüche 10 bis 25; und (b) ein zweites Arzneimittel.

**28.** Zusammensetzung nach einem der Ansprüche 10 bis 25 für die Verwendung in der Medizin.

**29.** Verwendung einer Zusammensetzung umfassend beides Interleukin-2 und Natriumdodecylsulfat für die Herstellung eines Medikamentes zur Verabreichung an einen Patienten, wobei IL-2 und Natriumdodecylsulfat in Form von Aggregaten vorliegen, und wobei die Zusammensetzung eines oder mehrere der folgenden Charakteristika hat:

(i) die Zusammensetzung hat eine spezifische Trübung ($\tau$) von weniger als 1,1 $cm^2/g$;
(ii) die Zusammensetzung enthält SDS/IL-2-Aggregate mit einem mittleren hydrodynamischen Durchmesser zwischen 8 nm und 20 nm;
(iii) die Zusammensetzung enthält zwischen 95-250 $\mu$g SDS pro mg IL-2; und/oder
(iv) die Aggregate sind durch ein Verfahren nach einem der Ansprüche 1 bis 9 erhältlich.

**30.** Verwendung nach Anspruch 29, wobei das Medikament zur Behandlung von Krebs ist.

**31.** Verwendung nach Anspruch 30, wobei der Krebs ein Nierenzellkarzinom ist.

**32.** Verwendung nach Anspruch 31, wobei der Krebs ein Melanom ist.

**33.** Verwendung nach einem der Ansprüche 29 bis 32, wobei der Patient mit einem zweiten Arzneimittel vorbehandelt wurde.

**34.** Verwendung nach einem der Ansprüche 29 bis 32, wobei das Medikament gemeinsam mit einem zweitem Arzneimittel verabreicht wird.

**35.** Verwendung eines zweiten Arzneimittels für die Herstellung eines Kombinationsmedikaments zur Verabreichung an einen Patienten, wobei der Patient mit einer Zusammensetzung nach einem der Ansprüche 10 bis 25 vorbehandelt wurde.

**36.** Verwendung eines zweiten Arzneimittels für die Herstellung eines Kombinationsmedikaments zur Verabreichung an einen Patienten, wobei das Medikament gemeinsam mit einer Zusammensetzung nach einem der Ansprüche 10 bis 25 verabreicht wird.

**37.** Verwendung von (a) Mikroaggregaten von Interleukin-2 und Natriumdodecylsulfat wie in Anspruch 10 definiert, und (b) einem zweiten Arzneimittel für die Herstellung eines Kombinationsmedikaments, wobei die Mikroaggregate einen mittleren hydrodynamischen Durchmesser zwischen 8 nm und 20 nm haben.

**38.** Zusammensetzung nach Anspruch 26, wobei das zweite Arzneimittel ein monoclonarer Antikörper ist.

**39.** Zusammensetzung nach Anspruch 26, wobei das zweite Arzneimittel ein Antikörper ist, welcher ADCC bewirkt.

**40.** Zusammensetzung nach Anspruch 26, wobei das zweite Arzneimittel ausgewählt ist aus der Gruppe bestehend aus: einem Anti-CD20-Antikörper; einem Anti-CD40-Antikörper; einem Anti-Her2-Antikörper; einem Anti-EGFR-Antikörper; einem Anti-VEGF-Antikörper; einem Anti-CD52-Antikörper; einem Anti-CD33-Antikörper; einem H2-Rezeptor-Agonisten; Bacille Calmette-Guerin; Thalidomid; Lenalidomid; einem Proteasom-Inhibitor; einer Kombination aus Cyclophosphamid, Vincristin und Prednison; einer Kombination aus Cyclophosphamid, Hydroxydoxorubicin, Vincristin und Prednison; einem Cytokin; Interferon $\alpha$; Interferon $\gamma$; 5-Fluoruracil; einer antiretroviralen Verbindung; einem Tyrosinkinase-Inhibitor; und Decitabin.

**41.** Zusammensetzung nach Anspruch 40, wobei der H2-Rezeptor-Agonist Histamin oder ein pharmazeutisch verträgliches Salz von Histamin wie Histamindihydrochlorid ist.

**42.** Verwendung nach einem der Ansprüche 33 bis 37, wobei das zweite Arzneimittel ein monoclonarer Antikörper ist.

**43.** Verwendung nach einem der Ansprüche 33 bis 37, wobei das zweite Arzneimittel ein Antikörper ist, welcher ADCC bewirkt.

**44.** Verwendung nach einem der Ansprüche 33 bis 37, wobei das zweite Arzneimittel ausgewählt ist aus der Gruppe bestehend aus: einem Anti-CD20-Antikörper; einem Anti-CD40-Antikörper; einem Anti-Her2-Antikörper; einem Anti-EGFR-Antikörper; einem Anti-VEGF-Antikörper; einem Anti-CD52-Antikörper; einem Anti-CD33-Antikörper; einem H2-Rezeptor-Agonisten; Bacille Calmette-Guerin; Thalidomid; Lenalidomid; einem Proteasom-Inhibitor; einer Kombination aus Cyclophosphamid, Vincristin und Prednison; einer Kombination aus Cyclophosphamid, Hydroxydoxorubicin, Vincristin und Prednison; einem Cytokin; Interferon $\alpha$; Interferon $\gamma$; 5-Fluoruracil; einer antiretroviralen Verbindung; einem Tyrosinkinase-Inhibitor; und Decitabin.

**45.** Verwendung nach Anspruch 44, wobei der H2-Rezeptor-Agonist Histamin oder ein pharmazeutisch verträgliches Salz von Histamin wie Histamindihydrochlorid ist.

## Revendications

**1.** Procédé de préparation d'interleukine-2 à usage pharmaceutique, comprenant les étapes consistant à : (a) ajouter du dodécylsulfate de sodium (SDS) à une composition comprenant de l'IL-2 avec des ponts disulfures intramoléculaires, pour donner une concentration finale de SDS d'au moins 500 $\mu$g de SDS par mg d'IL-2 ; (b) éliminer tous les multimères d'IL-2 précipitée liés de manière covalente par filtration sur gel ; et (c) réduire la concentration de SDS pour éliminer une partie mais pas la totalité du SDS de la composition, pour donner une composition qui contient entre 95 et 250 $\mu$g de SDS par mg d'IL-2.

**2.** Procédé selon la revendication 1, comprenant les étapes consistant à : purifier l'IL-2 par RP-HPLC ; précipiter l'IL-2 ; ajouter le SDS à l'IL-2 précipitée, pour donner une concentration finale de SDS d'au moins 500 $\mu$g de SDS par mg d'IL-2 ; éliminer tous les multimères covalents d'IL-2 précipitée par filtration sur gel ; et réduire la concentration de SDS pour éliminer une partie mais pas la totalité du SDS de la composition, pour donner une composition qui contient entre 95 et 250 $\mu$g de SDS par mg d'IL-2.

**3.** Procédé selon l'une quelconque des revendications précédentes, dans lequel la concentration finale de SDS dans l'étape (a) est d'au moins 900 $\mu$g/mg.

**4.** Procédé selon l'une quelconque des revendications précédentes, dans lequel le produit de l'étape (c) a une concentration de SDS comprise entre 135 et 180 μg/mg.

**5.** Procédé de préparation d'une composition comprenant de l'interleukine-2 et du dodécylsulfate de sodium, dans lequel : (i) l'interleukine-2 et le dodécylsulfate de sodium dans la composition sont présents sous la forme d'agrégats ; et (ii) le procédé comprend les étapes consistant à (a) ajouter du dodécylsulfate de sodium à une composition comprenant de l'interleukine-2 avec des ponts disulfures intramoléculaires, dans lequel une quantité suffisante de dodécylsulfate de sodium est ajoutée pour donner de l'interleukine solubilisée monomère, (b) éliminer tous les multimères d'IL-2 précipitée liés de manière covalente par filtration sur gel, et (c) réduire la concentration de dodécylsulfate de sodium à un niveau où l'interleukine-2 forme des agrégats avec le dodécylsulfate de sodium, de sorte que la composition, après lyophilisation et reconstitution, a une turbidité spécifique (τ) inférieure à 1,1 $cm^2/g$.

**6.** Procédé selon la revendication 5, dans lequel les agrégats ont un diamètre hydrodynamique moyen compris entre 8 nm et 20 nm.

**7.** Procédé selon l'une quelconque des revendications précédentes, dans lequel la concentration de SDS est réduite par diafiltration.

**8.** Procédé selon l'une quelconque des revendications précédentes, comprenant en outre une étape de : ajout de mannitol.

**9.** Procédé selon l'une quelconque des revendications précédentes, comprenant en outre une étape de : lyophilisation.

**10.** Composition comprenant de l'interleukine-2 et du dodécylsulfate de sodium, dans laquelle l'IL-2 et le dodécylsulfate de sodium sont présents sous la forme d'agrégats, et dans laquelle la composition a une ou plusieurs des caractéristiques suivantes :

(i) la composition a une turbidité spécifique (τ) inférieure à 1,1 $cm^2/g$ ;
(ii) la composition contient des agrégats de SDS/IL-2 ayant un diamètre hydrodynamique moyen compris entre 8 nm et 20 nm ;
(iii) la composition contient entre 95 et 250 μg de SDS par mg d'IL-2 ; et/ou
(iv) les agrégats peuvent être obtenus par le biais du procédé selon l'une quelconque des revendications précédentes.

**11.** Composition lyophilisée comprenant de l'interleukine-2 et du dodécylsulfate de sodium, laquelle composition peut être reconstituée avec un milieu aqueux pour donner la composition selon la revendication 10.

**12.** Composition selon la revendication 10, dans laquelle la turbidité spécifique de la composition est inférieure à 0,7 $cm^2/g$.

**13.** Composition selon la revendication 12, dans laquelle la turbidité spécifique de la composition est comprise entre 0,3 $cm^2/g$ et 0,6 $cm^2/g$.

**14.** Composition selon l'une quelconque des revendications 10 à 13, laquelle composition comprend des micro-agrégats de SDS/IL-2 ayant un diamètre hydrodynamique compris entre 11 nm et 13 nm.

**15.** Composition selon l'une quelconque des revendications 10 à 14, laquelle composition comprend du mannitol à une concentration comprise entre 40 et 50 mg de mannitol par mg d'IL-2.

**16.** Composition selon l'une quelconque des revendications 10 à 15, dans laquelle l'IL-2 est une IL-2 humaine.

**17.** Composition selon l'une quelconque des revendications 10 à 16, dans laquelle l'IL-2 est une IL-2 des-alanyl-1.

**18.** Composition selon l'une quelconque des revendications 10 à 17, dans laquelle l'IL-2 est une IL-2 sérine-125.

**19.** Composition selon l'une quelconque des revendications 10 à 18, dans laquelle l'IL-2 est l'IL-2 des-alanyl-1, sérine-125 humaine.

**20.** Composition selon l'une quelconque des revendications 10 à 19, dans laquelle l'IL-2 a la séquence d'acides aminés SEQ ID N° : 1.

**21.** Composition selon l'une quelconque des revendications 10 à 20, dans laquelle l'IL-2 est non glycosylée.

**22.** Composition selon l'une quelconque des revendications 10 à 21, dans laquelle l'IL-2 est purifiée après expression dans un hôte procaryote recombinant.

**23.** Composition selon l'une quelconque des revendications 10 à 22, avec une concentration d'IL-2 d'environ 1,1 mg/ml.

**24.** Composition lyophilisée selon la revendication 11 qui, lorsqu'elle est reconstituée avec 1,2 ml d'eau stérile pour injection, contient dans chaque millilitre : 1,1 mg d'IL-2 des-alanyl-2, sérine-125 humaine non glycosylée ; 50 mg de mannitol ; 0,18 mg de dodécylsulfate de sodium (SDS) ; 0,17 mg de phosphate de sodium monobasique ; et 0,89 mg de phosphate de sodium dibasique à pH 7,5, dans laquelle l'IL-2 et le SDS sont sous la forme d'agrégats de sorte que la composition reconstituée a une turbidité spécifique ($\tau$) inférieure à 1,1 $cm^2/g$.

**25.** Composition pharmaceutique aqueuse, pouvant être obtenue en reconstituant la composition lyophilisée selon la revendication 24 avec 1,2 ml d'eau stérile pour injection.

**26.** Composition selon l'une quelconque des revendications 10 à 25, et deuxième agent pharmaceutique, destinés à une administration simultanée séparée ou séquentielle.

**27.** Kit comprenant (a) la composition selon l'une quelconque des revendications 10 à 25 ; et (b) un deuxième agent pharmaceutique.

**28.** Composition selon l'une quelconque des revendications 10 à 25, destinée à être utilisée en médecine.

**29.** Utilisation d'une composition comprenant à la fois de l'interleukine-2 et du dodécylsulfate de sodium, dans la fabrication d'un médicament destiné à être administré à un patient, dans laquelle l'IL-2 et le dodécylsulfate de sodium sont présents sous la forme d'agrégats, et dans laquelle la composition a une ou plusieurs des caractéristiques suivantes :

(i) la composition a une turbidité spécifique ($\tau$) inférieure à 1,1 $cm^2/g$ ;
(ii) la composition contient des agrégats de SDS/IL-2 ayant un diamètre hydrodynamique moyen compris entre 8 nm et 20 nm ;
(iii) la composition contient entre 95 et 250 $\mu$g de SDS par mg d'IL-2 ; et/ou
(iv) les agrégats peuvent être obtenus par le biais du procédé selon l'une quelconque des revendications 1 à 9.

**30.** Utilisation selon la revendication 29, dans laquelle le médicament est destiné au traitement du cancer.

**31.** Utilisation selon la revendication 30, dans laquelle le cancer est l'hypernéphrome.

**32.** Utilisation selon la revendication 30, dans laquelle le cancer est le mélanome.

**33.** Utilisation selon l'une quelconque des revendications 29 à 32, dans laquelle le patient a été prétraité avec un deuxième agent pharmaceutique.

**34.** Utilisation selon l'une quelconque des revendications 29 à 32, dans laquelle le médicament est co-administré avec un deuxième agent pharmaceutique.

**35.** Utilisation d'un deuxième agent pharmaceutique, dans la fabrication d'un médicament de combinaison destiné à être administré à un patient, dans laquelle le patient a été prétraité avec la composition selon l'une quelconque des revendications 10 à 25.

**36.** Utilisation d'un deuxième agent pharmaceutique, dans la fabrication d'un médicament de combinaison destiné à être administré à un patient, dans laquelle le médicament est co-administré avec la composition selon l'une quelconque des revendications 10 à 25.

**37.** Utilisation (a) de micro-agrégats d'interleukine-2 et de dodécylsulfate de sodium tels que définis dans la revendication 10 et (b) d'un deuxième agent pharmaceutique, dans la fabrication d'un médicament de combinaison, dans laquelle les micro-agrégats ont un diamètre hydrodynamique moyen compris entre 8 nm et 20 nm.

**38.** Composition selon la revendication 26, dans laquelle le deuxième agent pharmaceutique est un anticorps monoclonal.

**39.** Composition selon la revendication 26, dans laquelle le deuxième agent pharmaceutique est un anticorps qui confère une ADCC.

**40.** Composition selon la revendication 26, dans laquelle le deuxième agent pharmaceutique est choisi dans le groupe constitué par : un anticorps anti-CD20 ; un anticorps anti-CD40 ; un anticorps anti-Her2 ; un anticorps anti-EGFR ; et un anticorps anti-VEGF ; un anticorps anti-CD52 ; un anticorps anti-CD33 ; un agoniste du récepteur H2 ; le bacille de Calmette et Guérin ; la thalidomide ; la lénalidomide ; un inhibiteur du protéasome ; une combinaison de cyclophosphamide, vincristine et prednisone ; une combinaison de cyclophosphamide, hydroxydoxorubicine, vincristine et prednisone ; une cytokine ; l'interféron $\alpha$; l'interféron $\gamma$; le 5-fluorouracile ; un composé anti-rétroviral ; un inhibiteur de la tyrosine kinase ; et la décitabine.

**41.** Composition selon la revendication 40, dans laquelle l'agoniste du récepteur H2 est l'histamine ou un sel pharmaceutiquement acceptable de l'histamine, tel que le dihydrochlorure d'histamine.

**42.** Utilisation selon l'une quelconque des revendications 33 à 37, dans laquelle le deuxième agent pharmaceutique est un anticorps monoclonal.

**43.** Utilisation selon l'une quelconque des revendications 33 à 37, dans laquelle le deuxième agent pharmaceutique est un anticorps qui confère une ADCC.

**44.** Utilisation selon l'une quelconque des revendications 33 à 3.7, dans laquelle le deuxième agent pharmaceutique est choisi dans le groupe constitué par : un anticorps anti-CD20 ; un anticorps anti-CD40 ; un anticorps anti-Her2; un anticorps anti-EGFR ; et un anticorps anti-VEGF; un anticorps anti-CD52 ; un anticorps anti-CD33 ; un agoniste du récepteur H2 ; le bacille de Calmette et Guérin ; la thalidomide ; la lénalidomide ; un inhibiteur du protéasome ; une combinaison de cyclophosphamide, vincristine et prednisone ; une combinaison de cyclophosphamide, hydroxydoxorubicine, vincristine et prednisone ; une cytokine ; l'interféron $\alpha$; l'interféron $\gamma$; le 5-fluorouracile ; un composé anti-rétroviral ; un inhibiteur de la tyrosine kinase; et la décitabine.

**45.** Utilisation selon la revendication 44, dans laquelle l'agoniste du récepteur H2 est l'histamine ou un sel pharmaceutiquement acceptable de l'histamine, tel que le dihydrochlorure d'histamine.

*FIGURE 1*

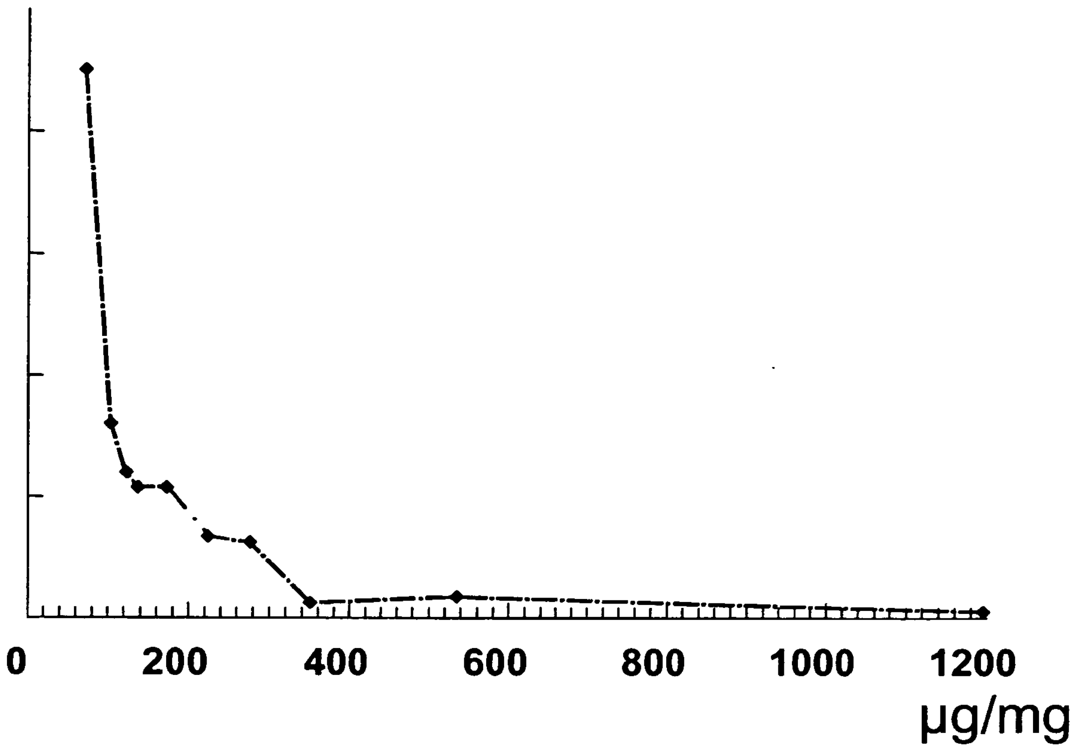


*FIGURE 15*

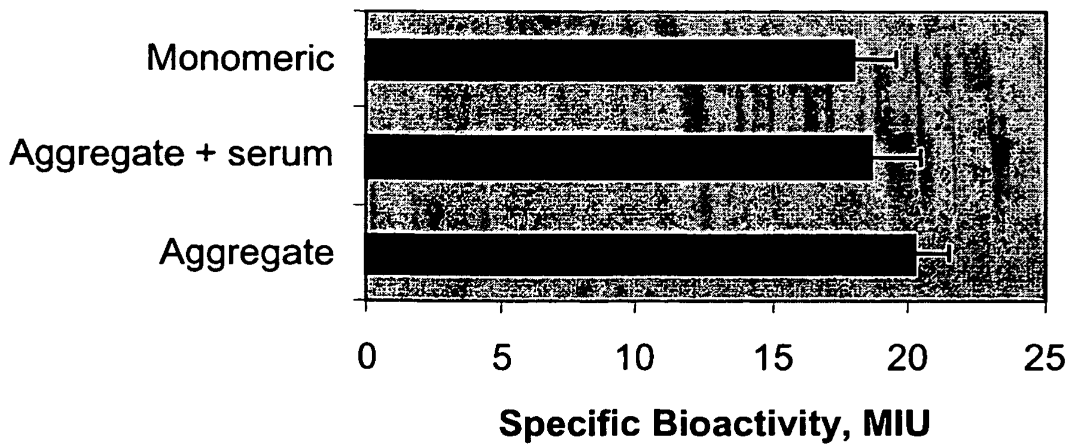

## *FIGURE 2*

ProThrSerSerSerThrLysLysThrGlnLeuGlnLeuGlu
HisLeuLeuLeuAspLeuGlnMetIleLeuAsnGlyIleAsn
AsnTyrLysAsnProLysLeuThrArgMetLeuThrPheLys
PheTyrMetProLysLysAlaThrGluLeuLysHisLeuGln
CysLeuGluGluGluLeuLysProLeuGluGluValLeuAsn
LeuAlaGlnSerLysAsnPheHisLeuArgProArgAspLeu
IleSerAsnIleAsnValIleValLeuGluLeuLysGlySer
GluThrThrPheMetCysGluTyrAlaAspGluThrAlaThr
IleValGluPheLeuAsnArgTrpIleThrPheSerGlnSer
IleIleSerThrLeuThr

## *FIGURE 3*

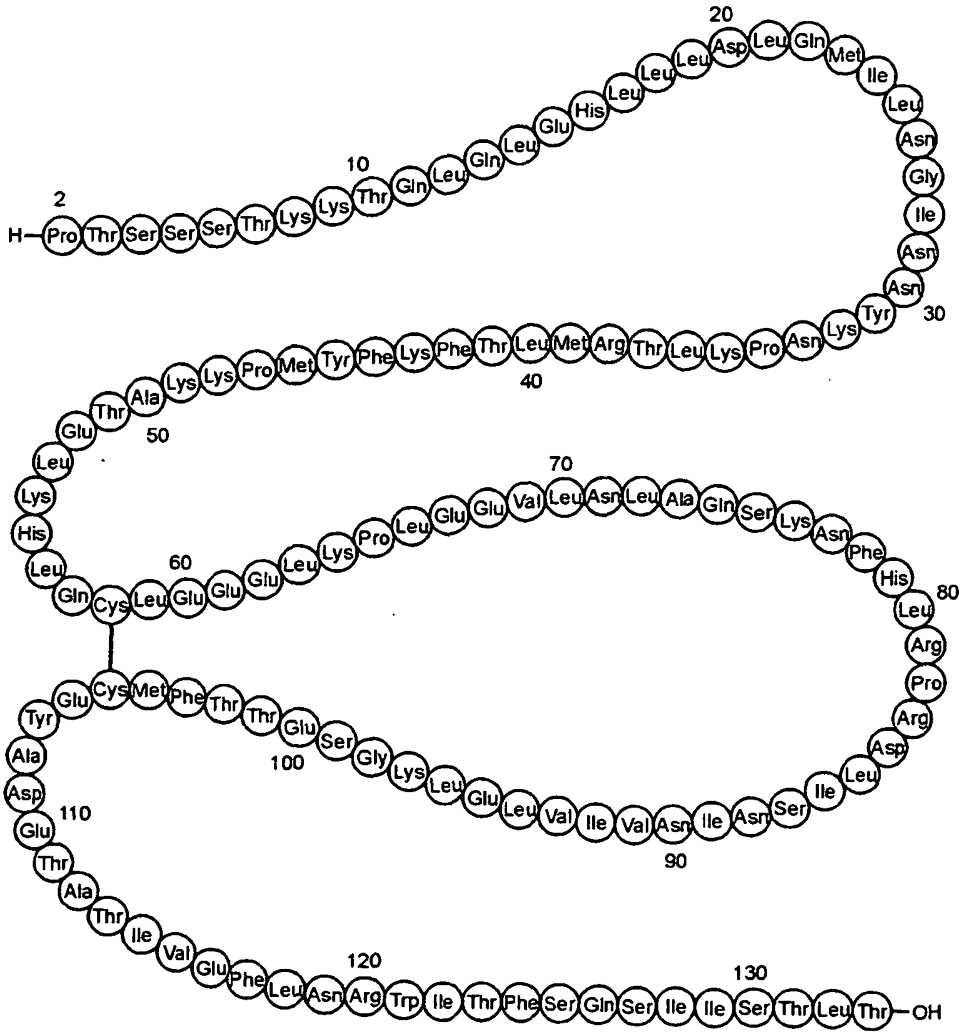

# *FIGURE 4*

```
        GCA
        ↓↓↓
1    ATG --- CCTACTTCAAGTTCTACAAAGAAAACACAGCTACAACTGGAGCATTTACTGCTG
     Met[Ala]ProThrSerSerSerThrLysLysThrGlnLeuGlnLeuGluHisLeuLeuLeu
     -1   1            5               10              15

58     GATTTACAGATGATTTTGAATGGAATTAATAATTACAAGAATCCCAAACTCACCAGGATG
       AspLeuGlnMetIleLeuAsnGlyIleAsnAsnTyrLysAsnProLysLeuThrArgMet
        20              25              30              35

118    CTCACATTTAAGTTTTACATGCCCAAGAAGGCCACAGAACTGAAACATCTTCAGTGTCTA
       LeuThrPheLysPheTyrMetProLysLysAlaThrGluLeuLysHisLeuGlnCysLeu
        40              45              50              55

178    GAAGAAGAACTCAAACCTCTGGAGGAAGTGCTAAATTTAGCTCAAAGCAAAAACTTTCAC
       GluGluGluLeuLysProLeuGluGluValLeuAsnLeuAlaGlnSerLysAsnPheHis
        60              65              70              75

238    TTAAGACCCAGGGACTTAATCAGCAATATCAACGTAATAGTTCTGGAACTAAAGGGATCT
       LeuArgProArgAspLeuIleSerAsnIleAsnValIleValLeuGluLeuLysGlySer
        80              85              90              95

298    GAAACAACATTCATGTGTGAATATGCTGATGAGACAGCAACCATTGTAGAATTTCTGAAC
       GluThrThrPheMetCysGluTyrAlaAspGluThrAlaThrIleValGluPheLeuAsn
       100             105             110             115

                       G   A                   A
                       ↓   ↓                   ↓
358    AGATGGATTACCTTTTCTCAGAGCATCATCTCAACACTGACTTGA
       ArgTrpIleThrPheSerGlnSerIleIleSerThrLeuThr***
       120             125             130
```

FIGURE 5

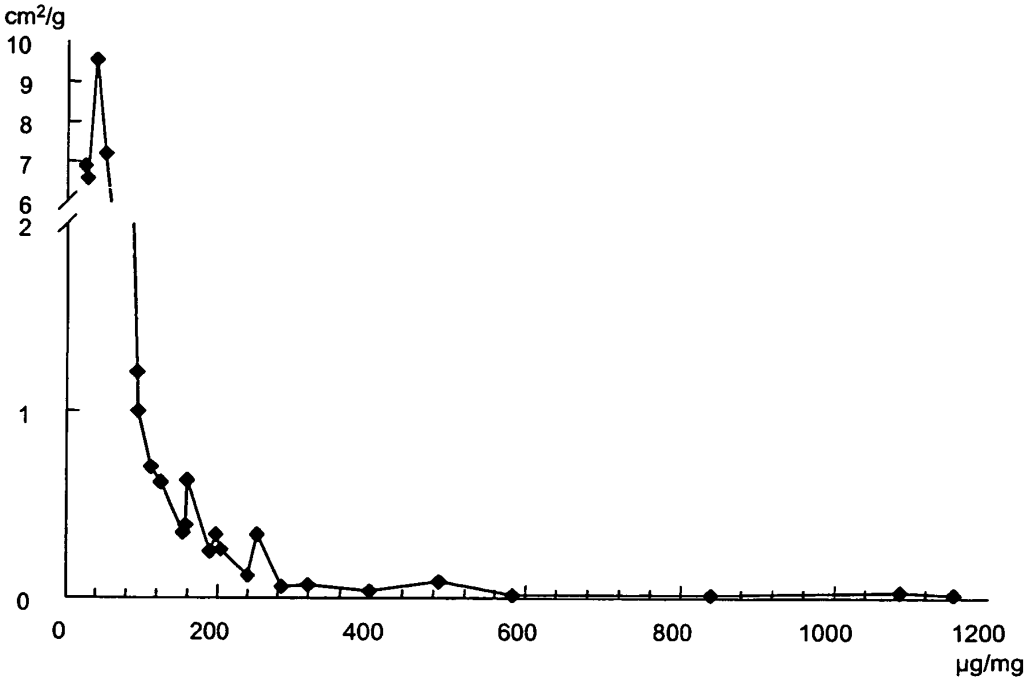


FIGURE 6

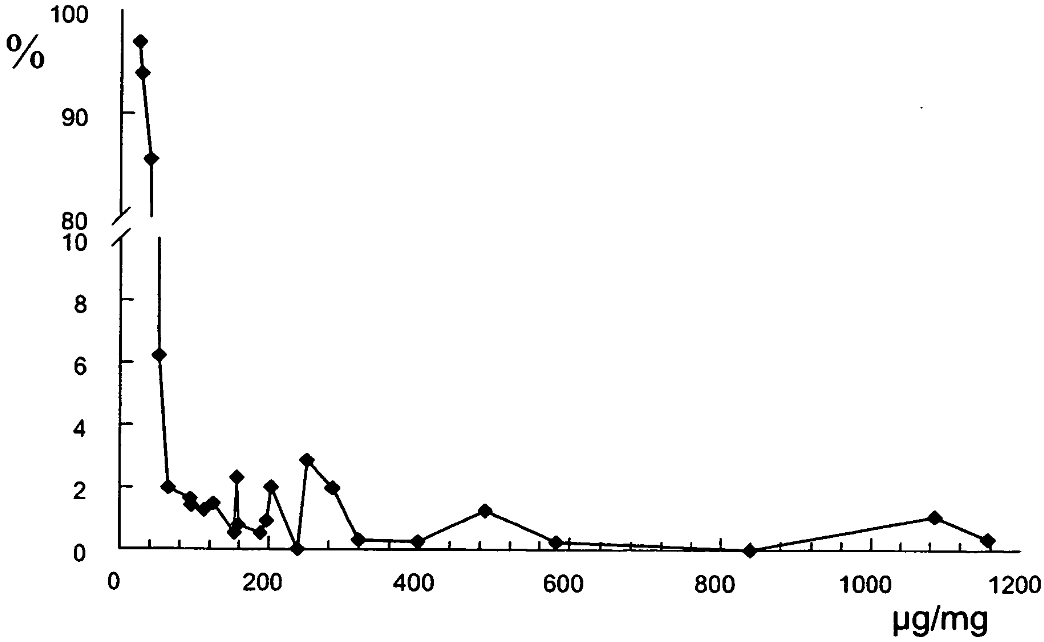

FIGURE 7

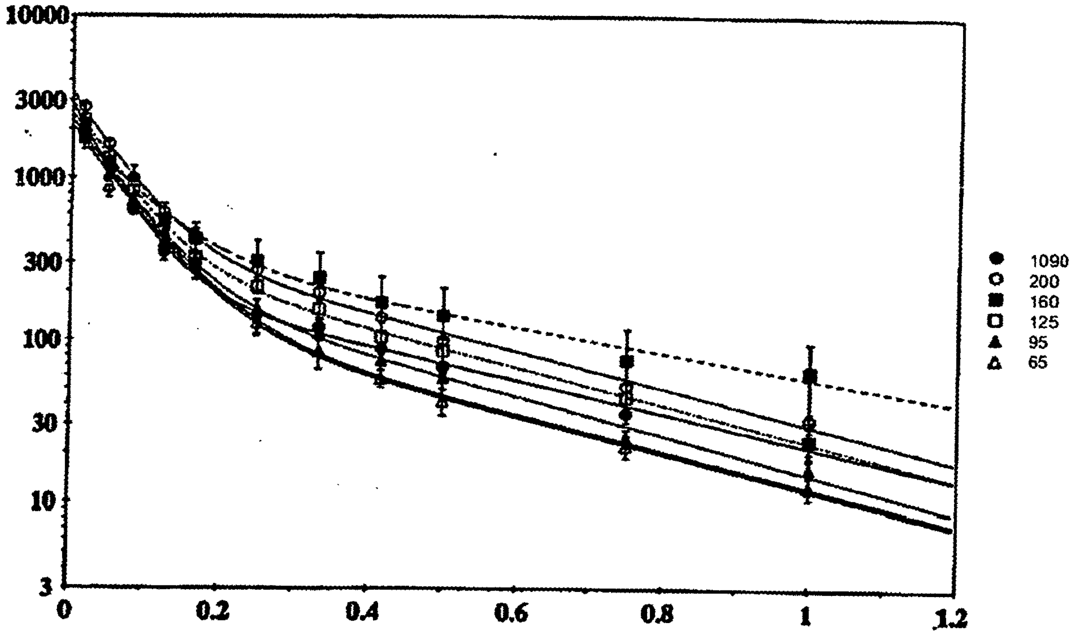


FIGURE 8

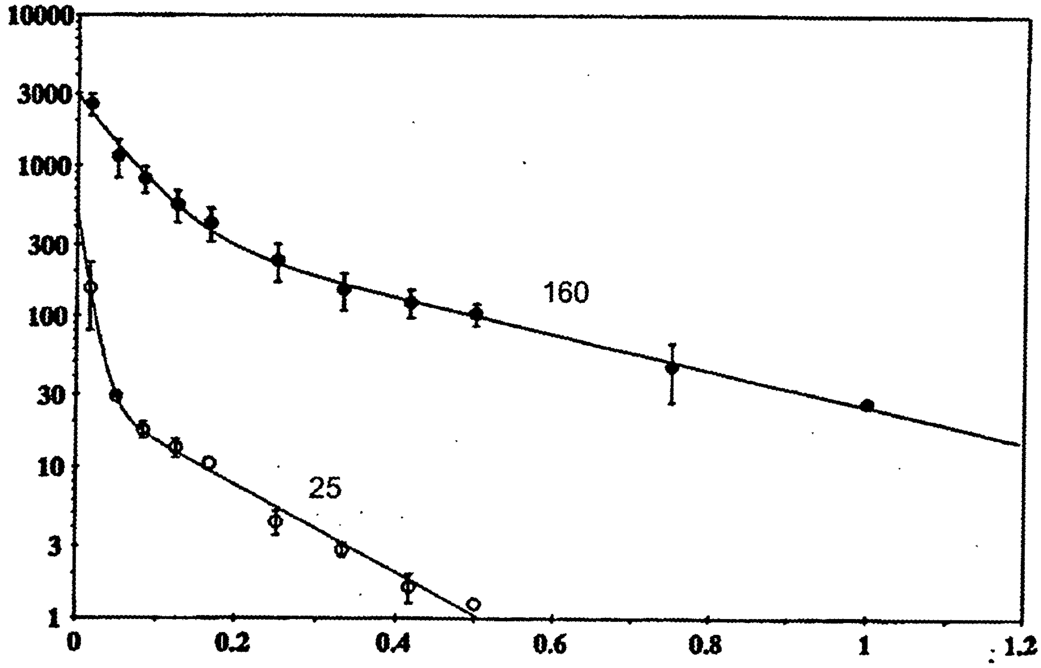

*FIGURE 9*

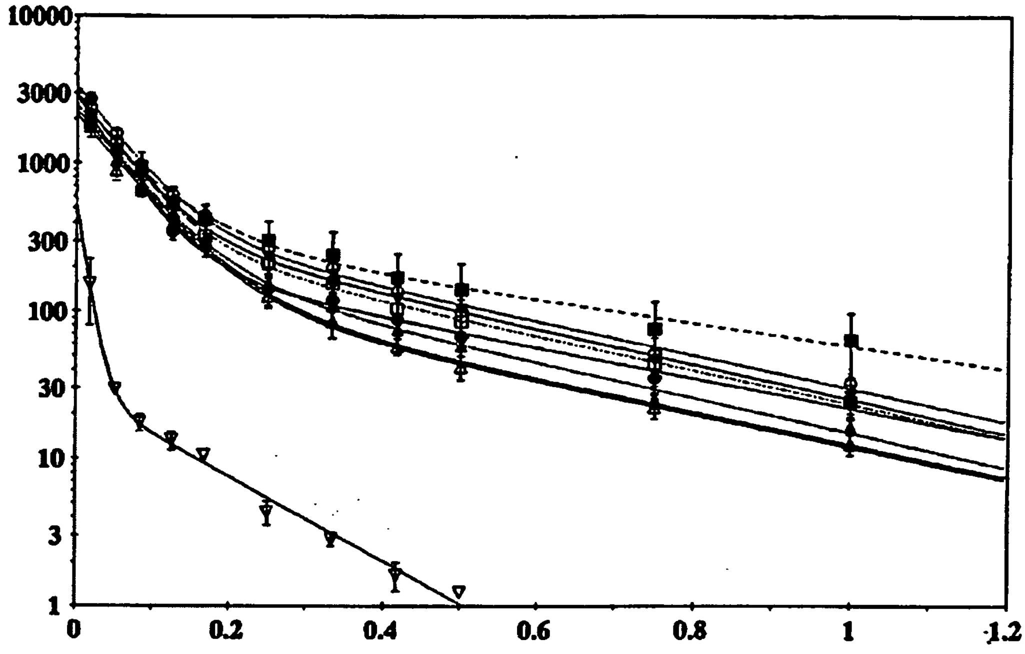


*FIGURE 10*

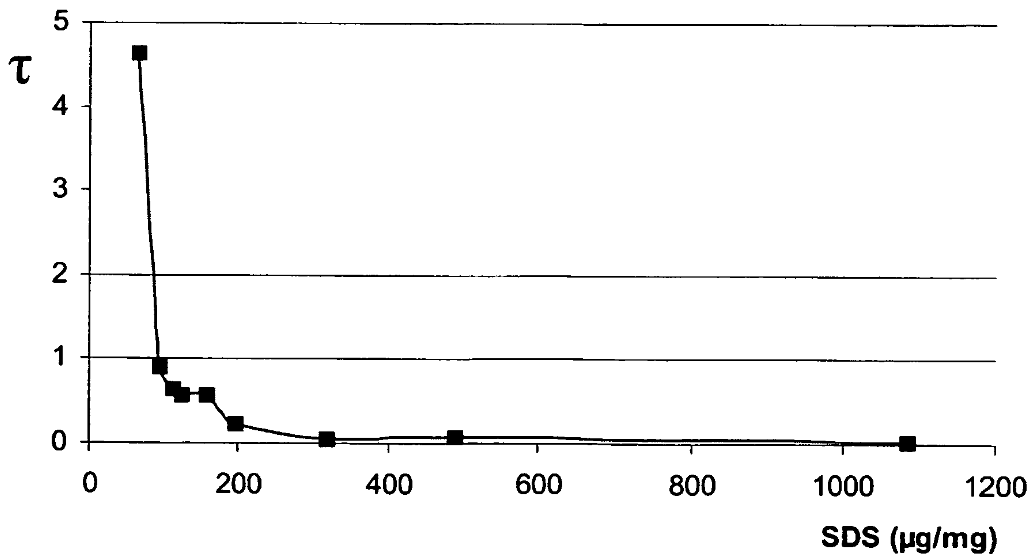

*FIGURE 11*

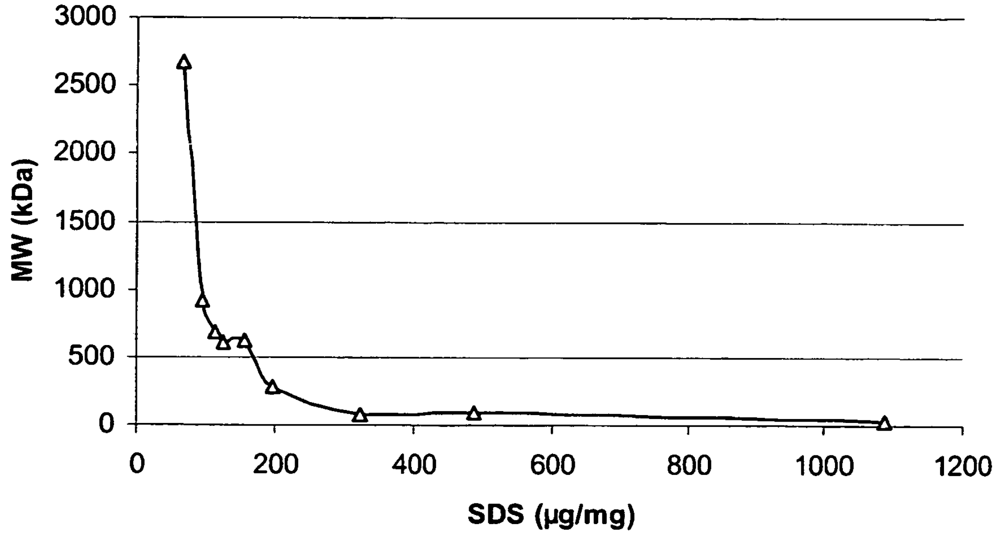


*FIGURE 12*

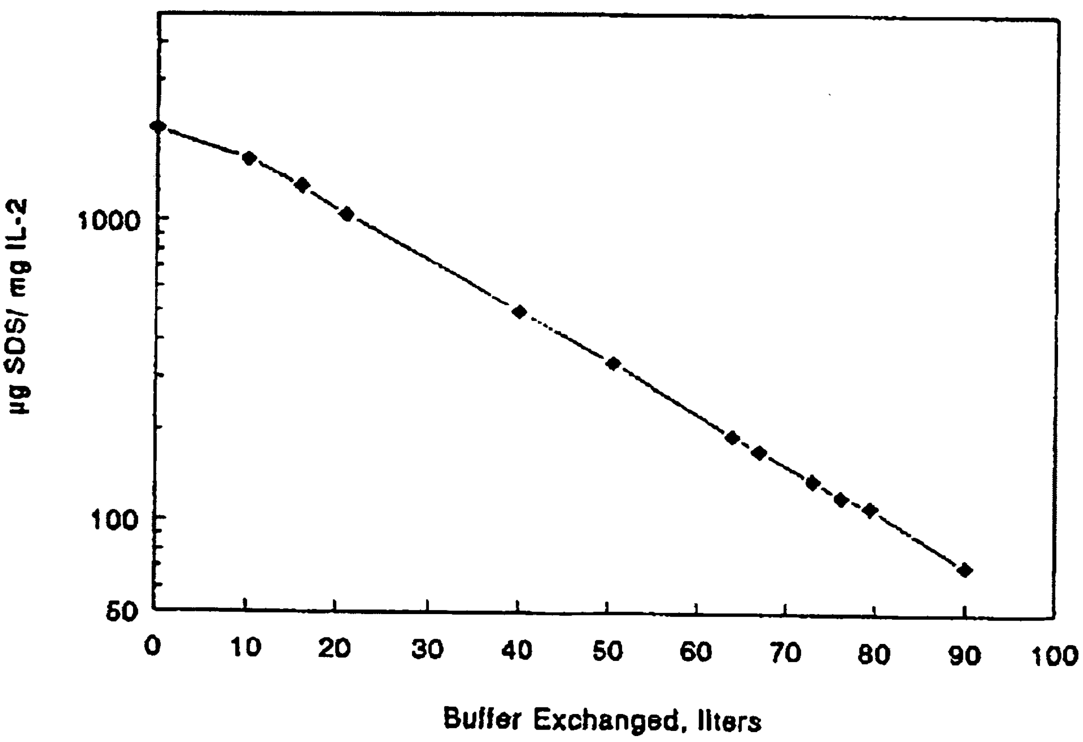

*FIGURE 13*

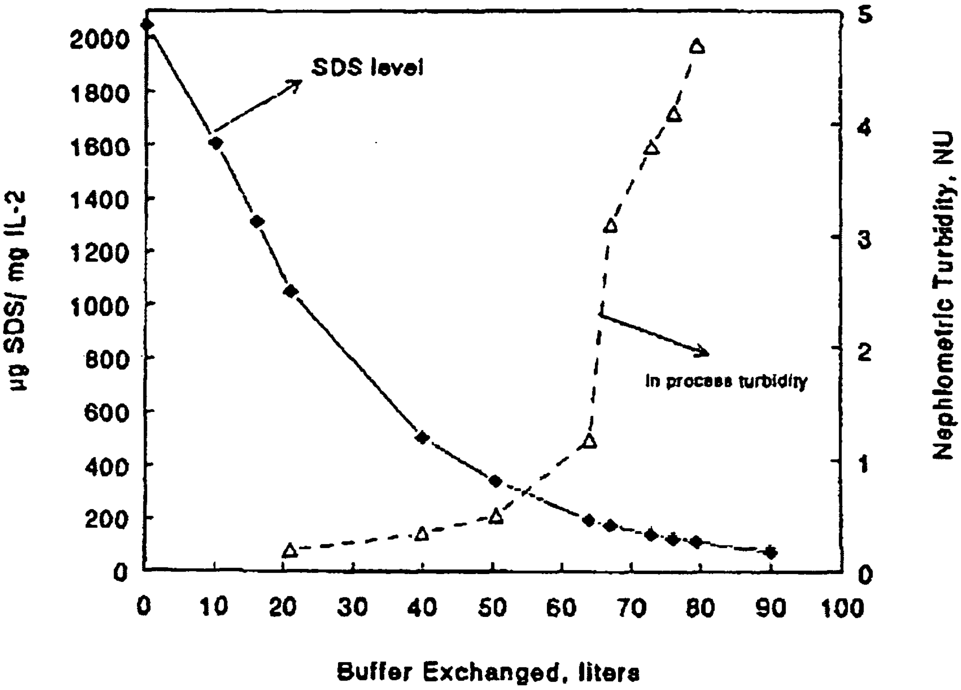


*FIGURE 14*

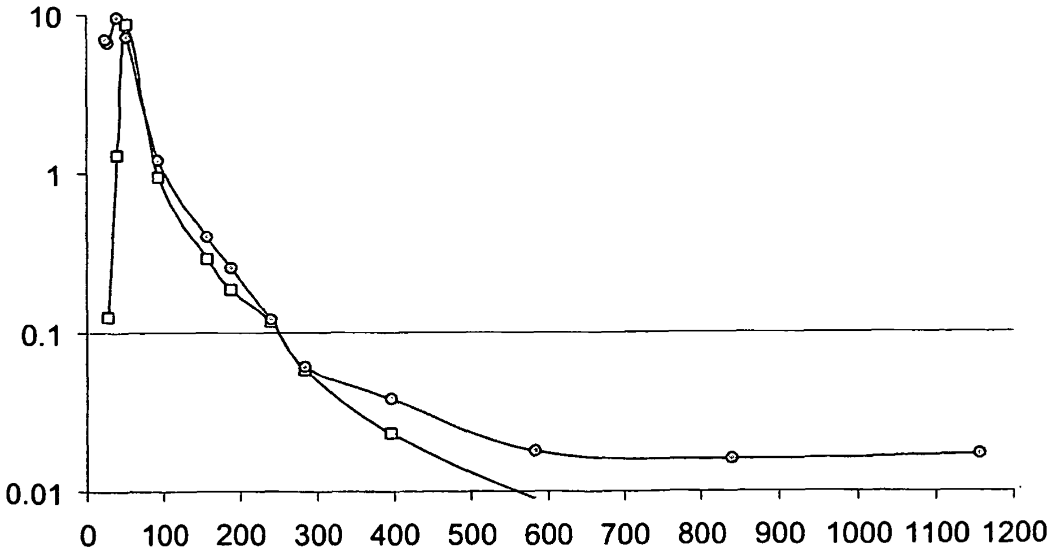

## *FIGURE 16*

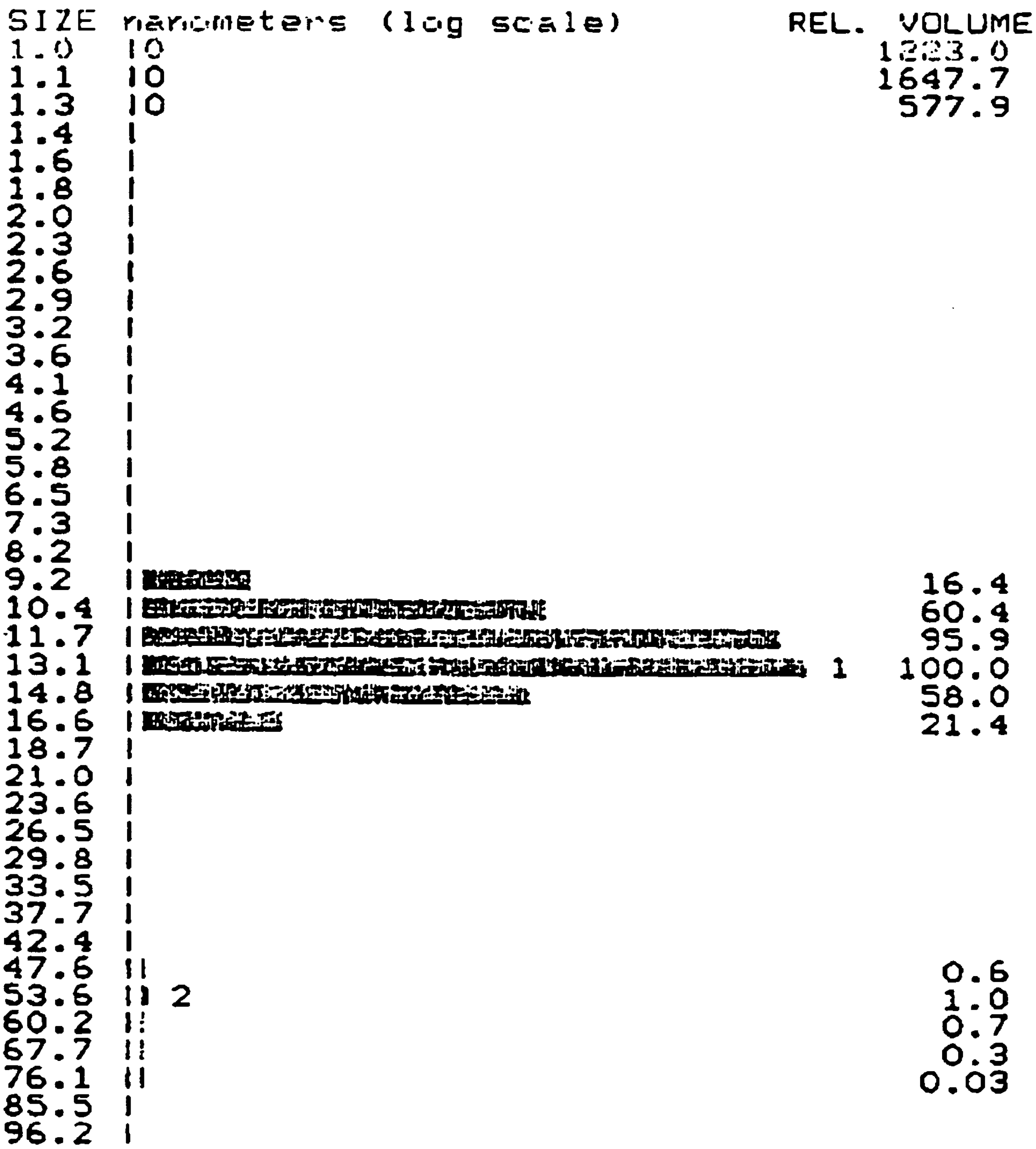
SIZE nanometers (log scale)
REL. VOLUME
1.0
1.1
1.3
1.4
1.6
1.8
2.0
2.3
2.6
2.9
3.2
3.6
4.1
4.6
5.2
5.8
6.5
7.3
8.2
9.2
10.4
11.7
13.1
14.8
16.6
18.7
21.0
23.6
26.5
29.8
33.5
37.7
42.4
47.6
53.6
60.2
67.7
76.1
85.5
96.2
1223.0
1647.7
577.9
16.4
60.4
95.9
1
100.0
58.0
21.4
2
0.6
1.0
0.7
0.3
0.03

## *FIGURE 17*

| SIZE | nanometers (log scale) | REL. NUMBER |
|---|---|---|
| 1.0 | 0 | 2036899.5 |
| 1.1 | 0 | 1931411.3 |
| 1.3 | | |
| 1.4 | | |
| 1.6 | | |
| 1.8 | | |
| 2.0 | | |
| 2.3 | | |
| 2.6 | | |
| 2.9 | | |
| 3.2 | | |
| 3.6 | | |
| 4.1 | | |
| 4.6 | | |
| 5.2 | | |
| 5.8 | | |
| 6.5 | | |
| 7.3 | | |
| 8.2 | | |
| 9.2 | | 34.6 |
| 10.4 | | 89.4 |
| 11.7 | 1 | 100.0 |
| 13.1 | | 73.4 |
| 14.8 | | 30.0 |
| 16.6 | | 7.8 |
| 18.7 | | |
| 21.0 | | |
| 23.6 | | |
| 26.5 | | |
| 29.8 | | |
| 33.5 | | |
| 37.7 | | |
| 42.4 | | |
| 47.6 | | 0.00 |
| 53.6 | 2 | 0.01 |
| 60.2 | | 0.00 |
| 67.7 | | 0.00 |
| 76.1 | | 0.00 |
| 85.5 | | |
| 96.2 | | |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EC 2057881
- US 4569790 A
- US 4604377 A
- WO 8504328 A
- US 4748234 A
- US 4908434 A
- US 4925919 A
- US 4853332 A
- US 5464939 A
- US 5824330 A
- US 4778879 A
- US 4992271 A
- US 5037644 A
- US 5078997 A
- US 4816440 A
- EP 0268110 B
- EP 0217645 B
- US 4738927 A
- US 4656132 A
- US 4530787 A
- US 4572798 A
- US 4931543 A
- US 4518584 A
- EP 0136489 A
- US 4752585 A
- EP 0200280 A
- WO 9960128 A
- US 5229109 A
- WO 0058456 A
- US 6168785 B


### Non-patent literature cited in the description


- **HORA et al.** *Biotechnology (N Y,* 1990, vol. 8, 755-8
- **MEYERS et al.** *Clin Pharmacol Ther.,* 1991, vol. 49, 307-13
- **HORA et al.** *Dev Biol Stand,* 1992, vol. 74, 295-306
- **HO et al.** *Vaccine,* 1992, vol. 10, 209-13
- **GEIGERT et al.** Stability and Characterization of Protein and Peptide Drugs: Case Histories. 1993
- **VEMURI.** *Dev Biol Stand.,* 1992, vol. 74, 341-51
- **ANDERSON et al.** *Am J Hosp Pharm,* 1992, vol. 49, 608-12
- **JOHNSTON et al.** *Pharm Res,* 1992, vol. 9, 425-34
- **ANDERSON et al.** *J Immunother,* 1992, vol. 12, 19-31
- **ANDERSON ; SORENSON.** *Clin Pharmacokinet,* 1994, vol. 27, 19-31
- **KONIGSBERG et al.** *Biochim Biophys Acta,* 1998, vol. 1370, 243-51
- **KANAOKA et al.** *J Pharm Pharmacol,* 2001, vol. 53, 295-302
- **OZBAS-TURAN et al.** *J Pharm Sci,* 2002, vol. 91, 1245-51
- **BERGMANN et al.** *Mol Immunol,* 1991, vol. 28 (1-2), 99-105
- **HEINIG ; VOGT.** *Electrophoresis,* 1999, vol. 20, 3311-28
- **NOMURA ; MURAKAMI.** *Bunseki Kagaku,* 1999, vol. 48, 111-6
- **FIELDEN ; CLAESSON.** *J Colloid Interface Sci,* 1998, vol. 198, 261-5
- **GABOR ; MAY.** Abstract # C-04, 1996. *FDA Science Forum Poster Abstracts,* 1996
- **SOKOLOFF ; FRIGON.** *Analytical Biochem,* 1981, vol. 118, 138-41
- **ARAKAWA et al.** *Int J Pept Protein Res,* 1994, vol. 43, 583-7
- **CAMPBELL ; DWEK.** Biological Spectroscopy
- **PICCORA.** Dynamic Light Scattering. Plenum Press, 1985
- **BAZAN.** *Science,* 1992, vol. 257, 410-2
- **WANG et al.** *Science,* 1984, vol. 224, 1431-3
- **MCKAY.** *Science,* 1992, vol. 257, 412
- **THEZE et al.** *Immunol Today,* 1996, vol. 17, 481-6
- **BUCHLI ; CIARDELLI.** *Arch Biochem Biophys,* 1993, vol. 307, 411-5
- **COLLINS et al.** *PNAS USA,* 1988, vol. 85, 7709-13
- **KUZIEL et al.** *J Immunol,* 1993, vol. 150, 5731
- **ECKENBERG et al.** *Cytokine,* 1997, vol. 9, 488-98
- **AHMAD et al.** *J Protein Chem,* 1994, vol. 13, 591-8
- **GEARING ; THORPE.** *J Immunol Methods,* 1988, vol. 114, 3-9

- **ROSSIO.** Cytokines and immune cell products. *Manual of Clinical and Laboratory Immunology,* 1997, 348-356
- **WADHWA et al.** Quantitative biological assays for individual cytokines. *Cytokine Cell Biology: A Practical Approach,* 2000, 207-239
- **SMITH ; WATERMAN.** *Adv. Appl. Math.,* 1981, vol. 2, 482-9

SEQUENCE LISTING

<110> CHIRON CORPORATION

<120> PREPARING ALDESLEUKIN FOR PHARMACEUTICAL USE

<130> P039597EP

<160> 5

<170> SeqWin99, version 1.02

<210> 1
<211> 132
<212> PRT
<213> Artificial Sequence

<220>
<223> Mature des-alanyl-1 serine-125 variant of human interleukin-2

<400> 1

```
Pro Thr Ser Ser Ser Thr Lys Lys Thr Gln Leu Gln Leu Glu His Leu
1               5                   10                  15

Leu Leu Asp Leu Gln Met Ile Leu Asn Gly Ile Asn Asn Tyr Lys Asn
            20                  25                  30

Pro Lys Leu Thr Arg Met Leu Thr Phe Lys Phe Tyr Met Pro Lys Lys
        35                  40                  45

Ala Thr Glu Leu Lys His Leu Gln Cys Leu Glu Glu Glu Leu Lys Pro
    50                  55                  60
```

Leu Glu Glu Val Leu Asn Leu Ala Gln Ser Lys Asn Phe His Leu Arg
65 70 75 80

Pro Arg Asp Leu Ile Ser Asn Ile Asn Val Ile Val Leu Glu Leu Lys
85 90 95

Gly Ser Glu Thr Thr Phe Met Cys Glu Tyr Ala Asp Glu Thr Ala Thr
100 105 110

Ile Val Glu Phe Leu Asn Arg Trp Ile Thr Phe Ser Gln Ser Ile Ile
115 120 125

Ser Thr Leu Thr
130

<210> 2
<211> 402
<212> DNA
<213> Artificial Sequence

<220>
<223> Des-alanyl-1 serine-125 variant of human interleukin-2

<400> 2

atgcctactt caagttctac aaagaaaaca cagctacaac tggagcattt actgctggat 60
ttacagatga ttttgaatgg aattaataat tacaagaatc ccaaactcac caggatgctc 120
acatttaagt tttacatgcc caagaaggcc acagaactga aacatcttca gtgtctagaa 180
gaagaactca aacctctgga ggaagtgcta aatttagctc aaagcaaaaa ctttcactta 240
agacccaggg acttaatcag caatatcaac gtaatagttc tggaactaaa gggatctgaa 300
acaacattca tgtgtgaata tgctgatgag acagcaacca ttgtagaatt tctgaacaga 360
tggattacct tttctcagag catcatctca acactgactt ga 402

<210> 3
<211> 133
<212> PRT
<213> Artificial Sequence

<220>
<223> Nascent des-alanyl-1 serine-125 variant of human interleukin-2

<400> 3

```
Met Pro Thr Ser Ser Ser Thr Lys Lys Thr Gln Leu Gln Leu Glu His
1               5                   10                  15

Leu Leu Leu Asp Leu Gln Met Ile Leu Asn Gly Ile Asn Asn Tyr Lys
            20                  25                  30

Asn Pro Lys Leu Thr Arg Met Leu Thr Phe Lys Phe Tyr Met Pro Lys
        35                  40                  45

Lys Ala Thr Glu Leu Lys His Leu Gln Cys Leu Glu Glu Glu Leu Lys
    50                  55                  60

Pro Leu Glu Glu Val Leu Asn Leu Ala Gln Ser Lys Asn Phe His Leu
65                  70                  75                  80

Arg Pro Arg Asp Leu Ile Ser Asn Ile Asn Val Ile Val Leu Glu Leu
                85                  90                  95

Lys Gly Ser Glu Thr Thr Phe Met Cys Glu Tyr Ala Asp Glu Thr Ala
            100                 105                 110

Thr Ile Val Glu Phe Leu Asn Arg Trp Ile Thr Phe Ser Gln Ser Ile
        115                 120                 125

Ile Ser Thr Leu Thr

        130
```

<210> 4
<211> 133
<212> PRT
<213> Homo sapiens

<400> 4

```
Ala Pro Thr Ser Ser Ser Thr Lys Lys Thr Gln Leu Gln Leu Glu His
1               5                   10                  15

Leu Leu Leu Asp Leu Gln Met Ile Leu Asn Gly Ile Asn Asn Tyr Lys
            20                  25                  30

Asn Pro Lys Leu Thr Arg Met Leu Thr Phe Lys Phe Tyr Met Pro Lys
        35                  40                  45

Lys Ala Thr Glu Leu Lys His Leu Gln Cys Leu Glu Glu Glu Leu Lys
    50                  55                  60

Pro Leu Glu Glu Val Leu Asn Leu Ala Gln Ser Lys Asn Phe His Leu
65                  70                  75                  80

Arg Pro Arg Asp Leu Ile Ser Asn Ile Asn Val Ile Val Leu Glu Leu
                85                  90                  95

Lys Gly Ser Glu Thr Thr Phe Met Cys Glu Tyr Ala Asp Glu Thr Ala
            100                 105                 110

Thr Ile Val Glu Phe Leu Asn Arg Trp Ile Thr Phe Cys Gln Ser Ile
        115                 120                 125

Ile Ser Thr Leu Thr


130
```

<210> 5
<211> 405
<212> DNA
<213> Artificial Sequence

<220>
<223> Human IL-2 sequence with Met in place of signal

<400> 5

```
atggcaccta cttcaagttc tacaaagaaa acacagctac aactggagca tttactgctg   60
gatttacaga tgattttgaa tggaattaat aattacaaga atcccaaact caccaggatg  120
ctcacattta agttttacat gcccaagaag gccacagaac tgaaacatct tcagtgtcta  180
gaagaagaac tcaaacctct ggaggaagtg ctaaatttag ctcaaagcaa aaactttcac  240
ttaagaccca gggacttaat cagcaatatc aacgtaatag ttctggaact aaagggatct  300
gaaacaacat tcatgtgtga atatgctgat gagacagcaa ccattgtaga atttctgaac  360
agatggatta ccttttgtca aagcatcatc tcaacactaa cttga                  405
```